# EUROPEAN PATENT APPLICATION

(11) **EP 1 563 886 A1**
(43) Date of publication of application: **17.08.2005**
(21) Application number: 05001365.5
(22) Date of filing: 24.01.2005
(51) Int. Cl.: B01D 15/08, B01J 47/00, B01L 3/00, C12N 15/10, G01N 35/02, G01N 30/02

(54) **Extraction system**

(30) Priority: 23.01.2004 JP 2004015089; 23.01.2004 JP 2004015090
(71) Applicant: Fuji Photo Film Co., Ltd., Kanagawa-ken (JP)
(72) Inventor: Fujimoto, Keiichi, Minamiashigara-shi Kanagawa-ken (JP); Torisawa, Nobuyuki, Minamiashigara-shi Kanagawa-ken (JP)
(74) Representative: Klunker . Schmitt-Nilson . Hirsch

(57) **Abstract**

An extraction cartridge (11) having a filter member is used to extract a specific material such as a nucleic acid. The specific material is caused to be adsorbed to the filter member by pressurizing a sample liquid containing a specific material. A pressurized-air supply mechanism (4) for introducing pressurized air into a plurality of the extraction cartridges (11) includes an air pump (43), opening and closing valves (45) for individually starting or stopping supply of the pressurized air to the plurality of extraction cartridges (11) and pressure relief valves (44a) for individually relieving the pressures in the extraction cartridges (11) to the atmosphere. These valves (45) and (44a) are disposed partway along a feed passage to the extraction cartridges. The extraction cartridges 11 are supplied with pressurized air, and then sealed. Upon completion of the drainage of any one of the extraction cartridges, the pressure relief valve (44a) associated with the completely drained extraction cartridge (11) is independently opened to relieve the pressurized air remaining therein.

## Description

### BACKGROUND OF THE INVENTION

### Field of the Invention

The present invention relates to an extraction system for extracting a specific material, for example, a nucleic acid, from a sample liquid by use of an extraction cartridge having therein a filter member. The present invention relates particularly to pressurization for such extraction performed by introducing pressurized air into the extraction cartridge.

### Description of the Related Art

Examples of known conventional extraction methods, for example, for extracting a nucleic acid, include those based on centrifugal action, magnetic beads or filters.

As a nucleic acid extraction system that uses a filter, there has been proposed a system for extracting a nucleic acid by loading a large number of filter tubes each having therein a filter to a tube rack; dispensing a sample liquid into the filter tubes; hermetically sealing the bottom of the rack along the periphery thereof via a sealing member such that an air chamber is formed; reducing the pressure within the rack, simultaneously applying suction to the respective filter tubes from the discharging side of the tubes so that the sample liquid is passed through the tubes and the nucleic acids are adsorbed to the filters; dispensing a washing liquid and an eluent to the filter tubes; and subjecting the filter tubes to suctioning in the same manner as described above to effect the washing and elution of the nucleic acids (see, for example, U.S. Patent No. 5,645,723).

This extraction system, however, suffers from the following problems. When the characteristics of the respective sample liquids are different from each other as in the case of drawn whole bloods, and when an extraction system, in which suction is totally applied to the filter tubes as disclosed in U.S. Patent No. 5,645,723, is used, once the suctioning of any of the filter tubes is completed and its or their resistance is lost, the suction force applied to the remaining filter tubes is reduced. Therefore, the processing for the sample liquids having higher viscosities may not be completed. Increasing the suctioning capacity, however, inhibits size reduction of the system, and additional time is required until a negative pressure is exerted due to the large suctioning volume. Further, it is difficult to detect that the liquid has been drained off completely and therefore a significant amount of time should be set for the detection. This limits enhancement of the processing efficiency. On the other hand, a sample liquid of lower viscosity is ejected with great force from the filter tubes and its frothy droplets are deposited onto and contaminate the adjacent filter tubes and the rack, thereby causing degradation in accuracy of the system.

When the filter tubes or filter cartridges are subjected to the aforementioned total vacuum suctioning, if any of the extraction cartridges are in the absence of air resistance because of defective injection of a sample liquid, defective loading to the system, or the like, normal operation cannot be ensured and it is also difficult to provide a mechanism for individually suctioning a large number of extraction cartridges.

In this regard, for the purpose of enhancing the processing efficiency and reducing the size of the system, there has been proposed another extraction system comprising an extraction cartridge which is provided with a filter member and functions to adsorb, wash, and collect a nucleic acid, wherein necessary solutions under pressure are passed through the filter member and drained into a waste liquid container or a collection container (see, for example, U.S. Patent Application Publication No. 20030170664).

In such an extraction system, in which pressurized air is supplied to the extraction cartridge for performing extraction under pressure, however, when a total amount of a solution under pressure passes through the filter member, the remaining pressurized air is violently expelled from a drain portion of the extraction cartridge together with the solution, whereby the effluent or drained liquid in the form of mist is blown away to contaminate the surrounding components. This may cause sample contamination.

Further, if pressurized air is introduced into a plurality of extraction cartridges by the operation of a single opening and closing valve, when the drainage of one of the extraction cartridge completes, the pressurized air leaks from this cartridge and thereby the pressurized air is insufficiently introduced into the remaining extraction cartridges and the internal pressure of each cartridge cannot be increased to reach a predetermined pressure. This causes the extraction condition to undesirably vary and results in insufficient extraction. As a result, reduction of the purity of nucleic acid, reduction of the reactivity in the course of the next process, and other undesirable effects are caused.

From this viewpoint, it is preferable to provide individual opening and closing valves, one for each extraction cartridge. However, providing a plurality of air pumps is disadvantageous in view of the compactness and cost. We focused on the fact that the drained liquid can be prevented from being blown off from the drain port of the cartridge, by relieving the pressurized air remaining in the extraction cartridge so as not to be violently expelled therefrom, upon completion of the drainage from the aforementioned extraction cartridge.

Meanwhile, we found that in the case where a plurality of the extraction cartridges are individually supplied with pressurized air, hermetically sealed, and a liquid is placed therein under pressure, it is important that introduction of pressurized air into a part of the extraction cartridges and pressure relief of another part of the extraction cartridges is enabled to be performed simultaneously, in order to ensure the measurement accuracy of the system. For example, to provide stable extraction, it is important that the conditions for introducing pressurized air into the individual extraction cartridges are kept invariant. An intended effect is achieved only if the pressure relief is carried out simultaneously with completion of the drainage of the liquid. However, all drainages for all of the extraction cartridges are not necessarily completed at the same time. If the pressure in the cartridge is relieved in the course of the liquid drainage, insufficient extraction is caused. This results in reduction of the purity of nucleic acid, reduction of the reactivity in the course of the next process, or other adverse effects. Therefore, it is desirable that the introduction of pressurized air and the pressure relief can be performed favorably for each extraction cartridge.

Further, the foregoing extraction system can provide effective adsorption and extraction of a nucleic acid, by controlling the flow rate of a sample solution containing a nucleic acid (a specific material) when the sample liquid passes through a filter member of the aforementioned extraction cartridge in the extraction process, to be within a predetermined range. To this end, it is required that the velocity and pressure of the pressurized air when the pressurized air enters the extraction cartridge are controlled to be within predetermined ranges, respectively.

Meanwhile, the discharging performance of the air pump for producing and feeding the pressurized air vary depending on product-to-product variation, temperature difference between warm weather and cold weather, time-lapse change, and so on. This induces the variation in the pressure rising property of the pressurized air to be introduced in the extraction cartridges, as a result of which the property of pressurizing the liquid within the extraction cartridges varies, and thereby a stable extraction cannot be achieved. Accordingly, it is desirable that the flow rate of the air fed by the air pump to the extraction cartridge is stabilized for better extraction.

### SUMMARY OF THE INVENTION

In view of the foregoing observations and description, an object of the invention is to provide an extraction system by which a specific material, such as a nucleic acid, contained in a sample liquid can be extracted efficiently in a short time.

In accordance with the present invention, there is provided a first extraction system for extracting a specific material, comprising:
an extraction cartridge provided with a filter member, wherein a sample liquid containing a specific material is injected into the extraction cartridge and the inside of the extraction cartridge is pressurized to cause the specific material within the sample liquid to be adsorbed to the filter member; and
a pressurized-air supply mechanism for introducing pressurized air into the extraction cartridge, the pressurized-air supply mechanism including: an air pump; a feed passage extending from the air pump to a plurality of extraction cartridges; a plurality of opening and closing valves, disposed partway along the feed passage and associated with the plurality of extraction cartridges in one-to-one correspondence, for individually starting or stopping the supply of the pressurized air to the plurality of extraction cartridges; and a plurality of pressure relief valves, disposed partway along the feed passage and associated with the plurality of extraction cartridges in one-to-one correspondence, for individually relieving the pressure within the plurality of extraction cartridges.

In accordance with the present invention, there is provided a second extraction system for extracting a nucleic acid, comprising:
an extraction cartridge provided with a filter member, wherein a sample liquid containing a nucleic acid is injected into the extraction cartridge, the inside of the extraction cartridge is pressurized to cause the nucleic acid within the sample liquid to be adsorbed to the filter member, a recovery liquid is subsequently dispensed into the extraction cartridges, the inside of the extraction cartridge is pressurized to cause the nucleic acid adsorbed to the filter member to be desorbed therefrom, and the nucleic acid is collected together with the recovery liquid; and
a pressurized-air supply mechanism for introducing pressurized air into the extraction cartridge, the pressurized-air supply mechanism including: an air pump; a feed passage extending from the air pump to a plurality of extraction cartridges; a plurality of opening and closing valves, disposed partway along the feed passage and associated with the plurality of extraction cartridges in one-to-one correspondence, for individually starting or stopping the supply of the pressurized air to the plurality of extraction cartridges; and a plurality of pressure relief valves, disposed partway along the feed passage and associated with the plurality of extraction cartridges in one-to-one correspondence, for individually relieving the pressure within the plurality of extraction cartridges.

It is preferable that the opening and closing valves are independently actuated to individually introduce the pressurized air into the plurality of the extraction cartridges, the plurality of the extraction cartridges are sealed, and upon completion of the drainage of any one of the extraction cartridges, the pressure relief valve associated with the completely drained extraction cartridge is independently opened to relieve the pressurized air remaining in the extraction cartridge.

It is also preferable that the pressurized-air supply mechanism further comprises a pressure sensor for individually detecting the internal pressure of the extraction cartridges, and the pressure relief valves are individually actuated depending on a detection result provided by the pressure sensor.

In accordance with the present invention, there is provided a third extraction system for extracting a specific material, comprising:
an extraction cartridge provided with a filter member, wherein a sample liquid containing a specific material is injected into the extraction cartridge and the inside of the extraction cartridge is pressurized to cause the specific material within the sample liquid to be adsorbed to the filter member; and
a pressurized-air supply mechanism for introducing pressurized air into the extraction cartridge, the pressurized-air supply mechanism including: an air pump; means for controlling air flow rate; and means for measuring air flow rate, wherein the means for controlling air flow rate is controlled depending on a measurement result from the means for measuring air flow rate, such that the flow rate of the air to be supplied to the extraction cartridge falls within a predetermined range.

In accordance with the present invention, there is provided a fourth extraction system for extracting a nucleic acid comprising:
an extraction cartridge provided with a filter member, wherein a sample liquid containing a nucleic acid is injected into the extraction cartridge, the inside of the extraction cartridge is pressurized to cause the nucleic acid within the sample liquid to be adsorbed to the filter member, a recovery liquid is subsequently dispensed into the extraction cartridge, the inside of the extraction cartridge is pressurized to cause the nucleic acid adsorbed to the filter member to be desorbed therefrom, and the nucleic acid is collected together with the recovery liquid; and
a pressurized-air supply mechanism for introducing pressurized air into the extraction cartridge, the pressurized-air supply mechanism including: an air pump; means for controlling air flow rate; and means for measuring air flow rate, wherein the means for controlling air flow rate is controlled depending on a measurement result from the means for measuring air flow rate, such that the flow rate of the air to be supplied to the extraction cartridge falls within a predetermined range.

It is preferable that the means for controlling air flow rate is a means for regulating the discharge rate from the air pump by drive-controlling the air pump, or a means for restricting the air flow rate at the suction side or discharge side of the air pump by the use of a flow rate control valve.

It is also preferable that the means for measuring air flow rate is a means for measuring a pressure in association with driving of the air pump in a closed circuit for air flow. It is also preferable that the air flow rate is corrected by driving the air pump at power-on, at the start of each extraction, or at predetermined timings.

With the foregoing first and second extraction systems, it is possible to provide a smaller mechanism for extracting a specific material such as a nucleic acid contained within a sample liquid by effectively and quickly performing an extraction operation by injecting a sample liquid containing a specific material such as a nucleic acid into a extraction cartridge provided with a filter member, and pressurizing the inside of the extraction cartridge to cause the specific material to be adsorbed to the filter member. Further, the pressurized-air supply mechanism used in the systems comprises a plurality of opening and closing valves, disposed partway along a feed passage to the plurality of the extraction cartridges and associated with the plurality of extraction cartridges in one-to-one correspondence, for individually starting or stopping supply of the pressurized air to the plurality of extraction cartridges; and a plurality of pressure relief valves, disposed partway along the feed passage and associated with the plurality of extraction cartridges in one-to-one correspondence, for individually relieving the pressure within the plurality of extraction cartridges. Therefore, introduction of pressurized air into a part of the extraction cartridges and pressure relief of other extraction cartridges can be performed simultaneously, as a result of which the measurement accuracy of the system can be ensured.

According to the foregoing systems, upon completion the drainage of any one of the extraction cartridges, the pressure relief valve associated with the completely drained extraction cartridge is independently opened to relieve the pressurized air remaining in the extraction cartridge. Therefore, the remaining pressurized air is prevented from being violently expelled from a drain portion of the extraction cartridge together with the liquid. Thus, blowing away of the drained liquid in the form of mist and sample contamination caused thereby can also be prevented. Further, for each extraction cartridge, introduction of pressurized air under optimum conditions and relief of the pressurized air at an optimum time are ensured, sample contamination is prevented, and a reliable extraction is provided. Accordingly, a process for extracting a specific material such as a nucleic acid can be stably performed, and reliability is enhanced without causing reduction of the purity of nucleic acid and reduction of the reactivity in the course of the next process.

Further, when the pressure relief valve is designed to be opened depending on a detection result provided by the pressure sensor, the pressure relief valve can be caused to be reliably opened upon completion of the drainage of the liquid from the cartridge associated therewith.

With the foregoing third and fourth extraction systems, it is possible to provide a smaller mechanism for extracting a specific material such as a nucleic acid contained within a sample liquid by effectively and quickly performing an extraction operation by injecting a sample liquid containing a specific material such as a nucleic acid into a extraction cartridge provided with a filter member, and pressurizing the inside of the extraction cartridge to cause the specific material to be adsorbed to the filter member.

Further, the pressurized-air supply mechanism is controlled depending on a measurement result from the means for measuring air flow rate, such that the flow rate of the air to be supplied to the extraction cartridge falls within a predetermined range. Therefore, the velocity and pressure of the pressurized air when the pressurized air enters the extraction cartridge are controlled to be within predetermined ranges, as a result of which, the property of pressurizing the liquid within the extraction cartridges hardly varies. Consequently, a stable extraction for extracting a specific material such as a nucleic acid by passing a sample liquid and the like through the filter member at a specified flow rate is achieved.

### BRIEF DESCRIPTION OF THE DRAWINGS

Figure 1 is a perspective view of a nucleic acid extraction system according to one embodiment of the invention with the cover thereof being removed;
Figure 2 is a schematic block diagram of the nucleic acid extraction system;
Figure 3 is a perspective view of a rack loaded in a holding mechanism of the nucleic acid extraction system;
Figure 4 is a perspective view showing the rack in operation;
Figure 5 is a diagram illustrating an air system of a pressurized air supply mechanism of the nucleic acid extraction system;
Figure 6 is a diagram illustrating a control system of the pressurized air supply mechanism;
Figure 7 is a flowchart diagram showing an exemplary routine of controlling a pressure applied to a sample;
Figure 8 is a flowchart diagram showing an exemplary routine of controlling a pressure applied to a washing liquid or a recovery liquid;
Figure 9 is a graphical representation showing the variations in the internal pressure of an extraction cartridge;
Figures 10A to 10G show processing steps for an extracting operation;
Figure 11 is a perspective view of the extraction cartridge;
Figure 12 shows a flowchart diagram illustrating an exemplary routine of calculating a correction amount used for correcting a discharge rate of an air pump;
Figure 13 shows a flowchart diagram illustrating another exemplary routine of calculating a correction amount used for correcting a discharge rate of an air pump;
Figure 14 shows a flowchart diagram illustrating an exemplary routine of correcting a discharge rate of an air pump; and
Figure 15 is a diagram illustrating a control system of the pressurized air supply mechanism of the extraction system according to the fourth embodiment of the invention.

### DESCRIPTION OF THE PREFERRED EMBODIMENTS

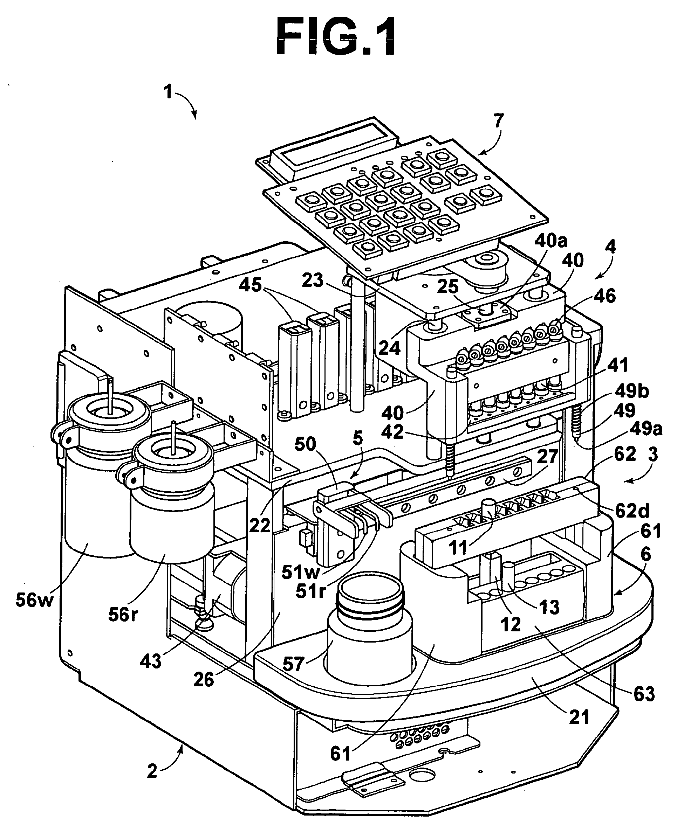

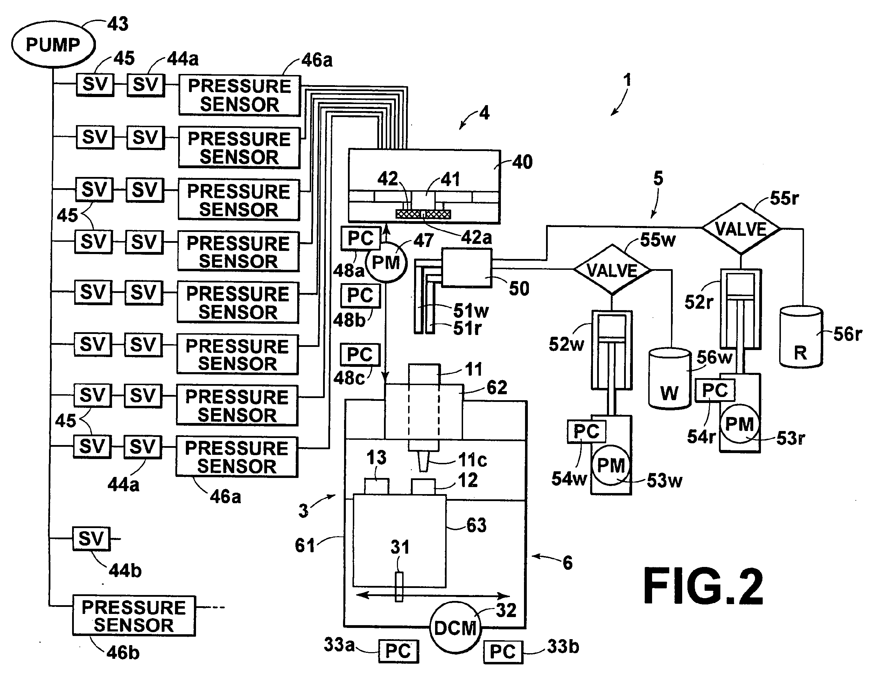

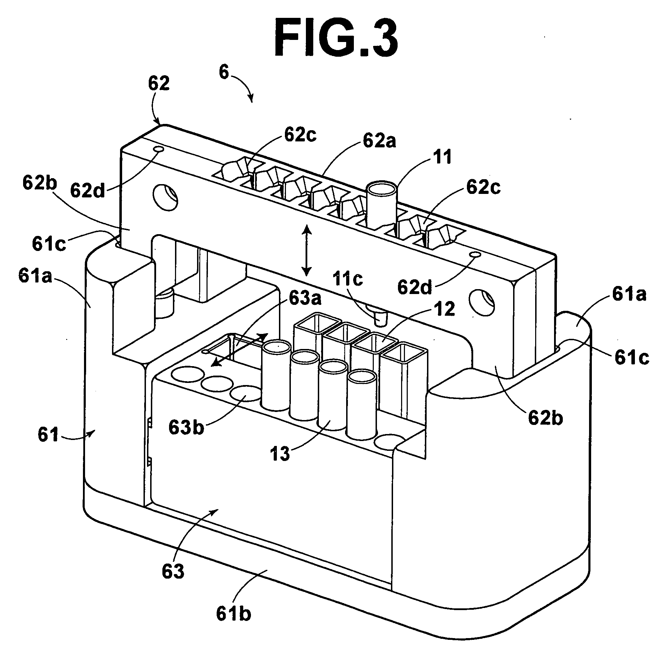

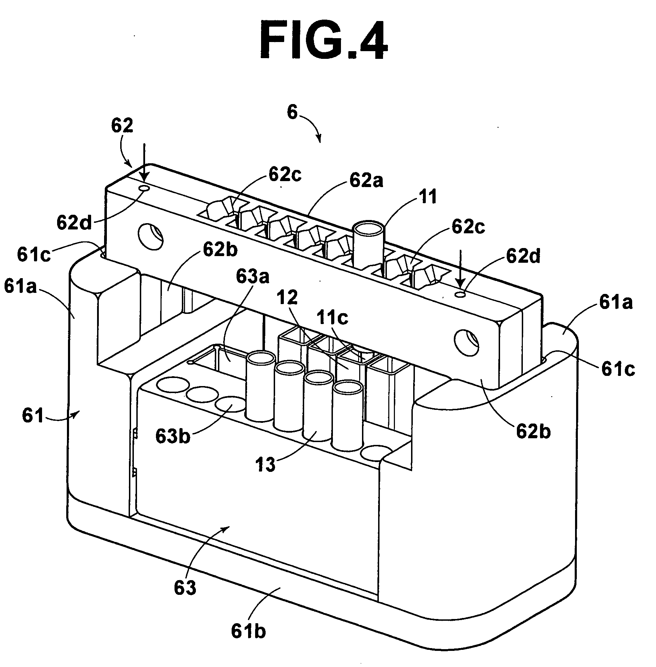

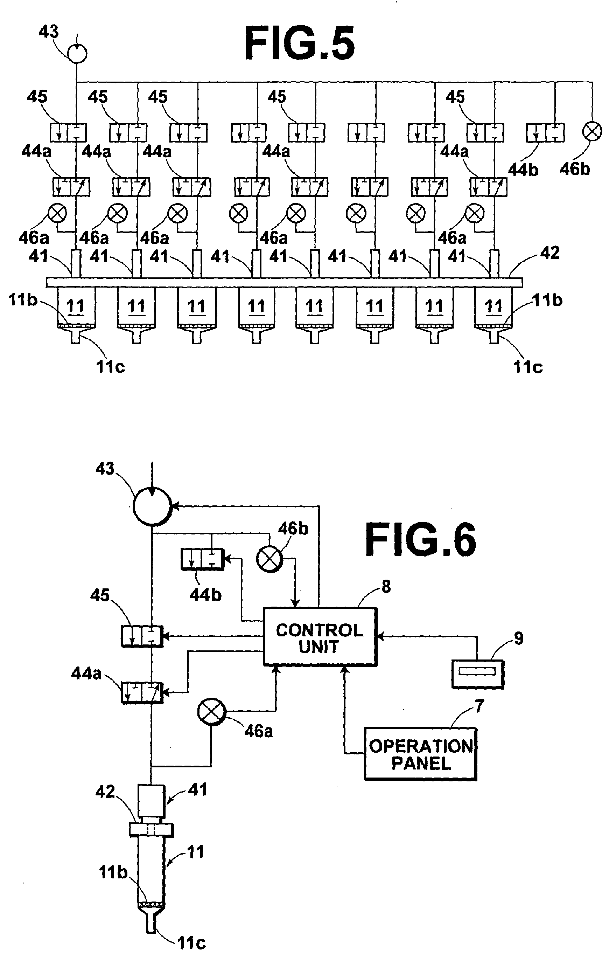

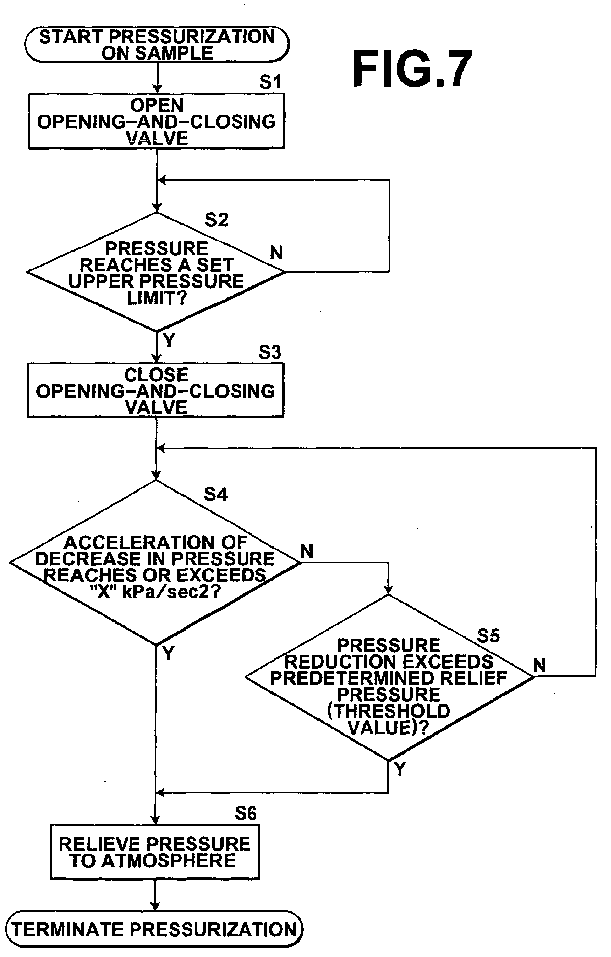

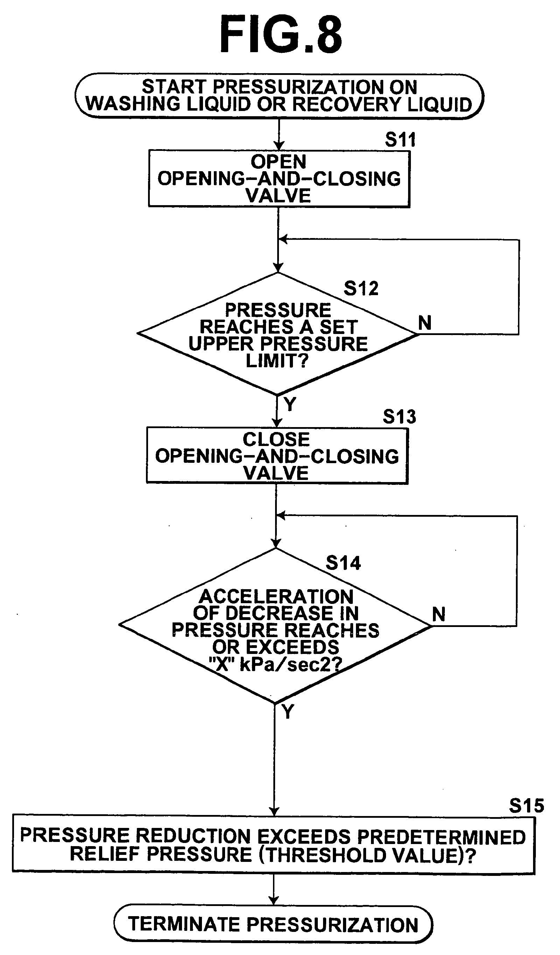

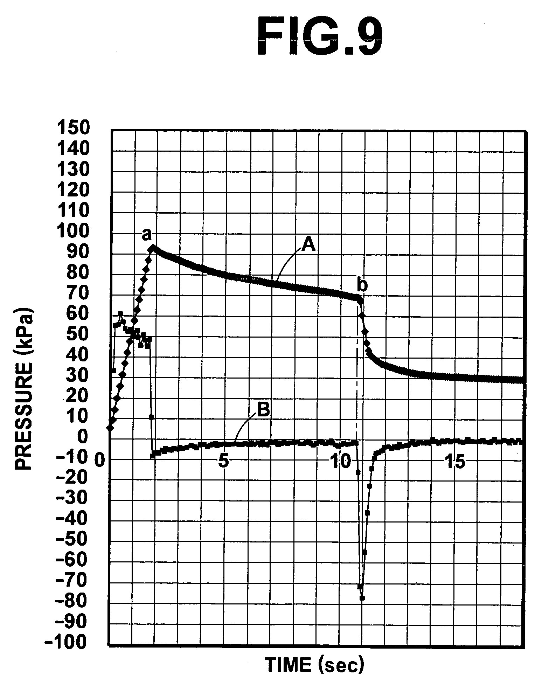

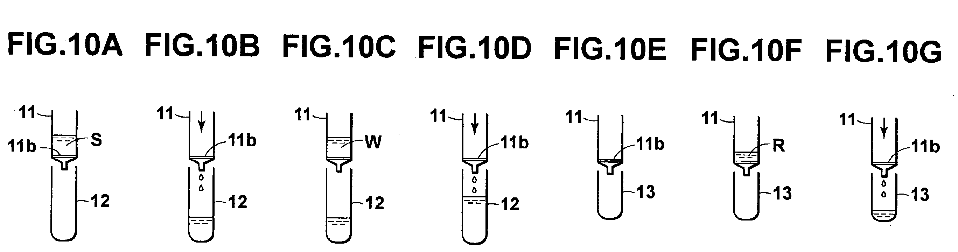

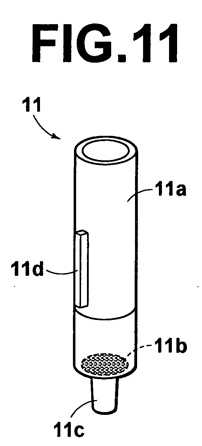

Hereinafter, an embodiment of the first and second extraction systems according to the present invention will be described in detail with reference to the drawings. Figure 1 is a perspective view of a nucleic acid extraction system according to an embodiment of the invention, which is provided as an representative example of the first and second extraction systems of the present invention, with the cover thereof being removed; Figure 2 is a schematic block diagram of the nucleic acid extraction system; Figure 3 is a perspective view of a rack loaded in a holding mechanism of the nucleic acid extraction system; Figure 4 is a perspective view showing the rack in operation; Figure 5 is a diagram illustrating an air system of a pressurized air supply mechanism of the nucleic acid extraction system; Figure 6 is a diagram illustrating a control system of the pressurized air supply mechanism; Figures 7 and 8 are flowchart diagrams respectively showing exemplary routines of controlling an applied pressure; Figure 9 is a graphical representation showing the variations in the internal pressure of an extraction cartridge; Figures 10A to 10G show processing steps for an extracting operation; and Figure 11 is a perspective view of the extraction cartridge.

Before addressing the features of a nucleic acid extraction system 1 according to the embodiment of the invention, which is provided as an representative example of the first and second extraction systems of the present invention, it should be noted that the nucleic acid extraction system 1 is that which extracts a nucleic acid as a specific material contained in a sample liquid by use of an extraction cartridge 11 (a filter cartridge) shown in Figure 11. This extraction cartridge 11 comprises a tubular body 11a having an open top. A filter member 11b is retained on the bottom of the tubular body 11a. A portion of the tubular body 11a extending below the filter member 11b is shaped in the form of a funnel, and a thin nozzle-like drain portion 11c, centrally disposed on the underside of the tubular body 11a, projects downward over a predetermined length. Projections 11d are formed on the axially opposite sides of the tubular body 11a such that each projection 11d projects radially outward and extends longitudinally. A sample liquid, a washing liquid and a recovery liquid (each of which will be described later) are individually dispensed through the upper opening of the extraction cartridge 11 and then pressurized air is introduced through the upper opening, whereby each liquid flows down through the filter member 11b and is discharged from the drain portion 11c into a waste liquid container 12 or a collection container 13 (both of which will be described later). In this particular embodiment, the tubular body 11a is composed of an upper part and a lower part which are engaged with each other.

In principle, the nucleic acid extraction system 1 extracts and purifies a nucleic acid according to an extraction procedure comprising the steps as shown in Figure 10A to Figure 10G. Hereinafter, the step shown in Figure 10A is referred to as step (a), the step shown in Figure 10B is referred to as step (b), and so on. First, in step (a), a sample liquid S containing the nucleic acid, which has been subjected to solubilization, is injected into the extraction cartridge 11 positioned above the waste liquid container 12. Then, in step (b), pressurized air is introduced into the extraction cartridge 11 so that the sample liquid S is passed through the cartridge via the filter member 11b, the nucleic acid is adsorbed to the filter member 11b, and the liquid component is drained through the cartridge 11 into the waste liquid container 12.

Next, in step (c), a washing liquid W is automatically dispensed in the extraction cartridge 11. In step (d), pressurized air is introduced in the extraction cartridge 11 to apply pressure thereto so that the impurities other than the nucleic acid are removed by washing with the nucleic acid being retained on the filter member 11b. The washing liquid W is drained through the cartridge into the waste liquid container 12. Steps (c) and (d) may be repeated.

After that, in step (e), the waste liquid container 12 positioned below the extraction cartridge 11 is replaced with the collection container 13, and in step (f), a recovery liquid R is automatically dispensed in the extraction cartridge 11, pressurized air is introduced into the extraction cartridge 11 to apply pressure thereto so that the binding force between the nucleic acid and the filter member 11b is weakened to separate the adsorbed nucleic acid therefrom, and the recovery liquid R, in which the nucleic acid is contained, is drained from the extraction cartridge 11 and collected into the collection container 13.

The filter member 11b in the extraction cartridge 11 described above is basically porous, to enable nucleic acid to pass therethrough, and includes a surface having a property of adsorbing a nucleic acid contained in a sample liquid with a chemical binding force. Thus, the filter member 11b is designed such that the adsorption of the nucleic acid is maintained when the filter member 11b undergoes a washing process using a washing liquid, while the adsorbing force is weakened and the nucleic acid is separated from the filter member 11b when the filter member 11b undergoes a collection process using a recovery liquid. As described in detail in U.S. Patent Application Publication No. 20030170664 regarding a method of isolating and purifying nucleic acid, the foregoing filter member 11b, for example, may be composed of organic high polymers having a hydroxyl group on a surface thereof. Surface-saponified cellulose acetate is preferred as the organic high polymer having a hydroxyl group on its surface. Any cellulose acetate such as cellulose monoacetate, cellulose diacetate, and cellulose triacetate may be used, but in particular, cellulose triacetate is preferable. The surface that comes in contact with saponification treatment solution (e.g., NaOH) is saponified, while the structure remains cellulose acetate. This allows the amount of hydroxyl group (density) on the surface to be controlled according to the degree of surface saponification (surface saponification degree). The adsorbing effect of nucleic acids increases as a larger number of hydroxyl groups are present. For example, in the case of cellulose acetate such as cellulose triacetate, the surface-saponification ratio is preferably 5% or more, more preferably 10% or more. Cellulose acetate is preferably provided in the form of a porous membrane.

The "sample liquid S containing nucleic acids" is a solution which is prepared by adding an aqueous organic solvent to a solution obtained by subjecting a sample containing a cell or a virus to the solubilization. For example, in the field of diagnostics, the sample liquids S include: body fluids collected as samples, such as whole blood, plasma, serum, urine, stool, sperm, and saliva; plants (or a part thereof); animals (or a part thereof); and solutions prepared from biological materials such as lysates and homogenates of the above samples. The "solubilization" is a treatment for treating a sample with an aqueous solution containing a reagent capable of dissolving cell membranes and solubilizing nuclear membranes (for example, a solution comprising a guanidine salt, a surfactant and a protease). For example, when a sample is whole blood, erythrocytes and various proteins are broken and depolymerized in order to prevent their non-specific adsorption to the filter member 11b and clogging thereof, and lysis of leukocytes and nuclear membranes is carried out to solubilize the nucleic acids which are to be extracted. The "water-soluble organic solvents" to be used herein include ethanol, isopropanol, and propanol. Among these, ethanol is preferable. The concentration of the water-soluble organic solvent is preferably 5% by weight to 90% by weight, more preferably 20% by weight to 60% by weight. It is particularly preferable that ethanol is added so as to have as high a concentration as possible, but to such an extent that aggregation does not occur.

The "washing liquid W" serves to wash out impurities which were present in the sample liquid and were adsorbed onto the filter member 11b with the nucleic acids. This liquid has a composition by which only impurities are desorbed from the filter member 11b with the nucleic acids remaining adsorbed. The washing liquid W is a solution containing a base agent and a buffer agent, and optionally a surfactant. The base agents include aqueous solutions having approximately 10 to 100% by weight (preferably approximately 20 to 100% by weight, more preferably approximately 40 to 80% by weight) of methanol, ethanol, isopropanol, n-isopropanol, butanol, actone, and the like.

The "recovery liquid R" has preferably a low salt concentration, and more preferably a solution having a salt concentration of 0.5 M or lower is used. Examples of the liquid to be used include purified distilled water and TE buffers.

As shown in Figures 1 and 2, the nucleic acid extraction system 1 comprises: a main body 2 of the nucleic acid extraction system 1; a holding mechanism 3 for holding a plurality of extraction cartridges 11, a plurality of waste liquid containers 12, and a plurality of collection containers 13 in the main body 2; a pressurized-air supply mechanism 4 for introducing pressurized air into the extraction cartridge 11; and a dispensing mechanism 5 for dispensing a washing liquid W and a recovery liquid R into the extraction cartridge 11; etc. In the following, each mechanism 3 to 5 will be specifically described.

### <Holding Mechanism>

The holding mechanism 3 comprises a holding stage 21 disposed at a front lower part of the main body 2. A rack 6, in which the extraction cartridges 11, the waste liquid containers 12, and the recovery containers 13 are held, is mounted on the holding stage 21. As also shown in Figure 3, the rack 6 comprises a stand 61, a cartridge holder 62, and a container holder 63.

The stand 61 comprises opposite columns 61a which cooperate to support the cartridge holder 62 in a manner to be movable up and down, and a bottom plate 61b that is disposed between the columns 61a and supports thereon the container holder 63 in a manner so as to be movable back and forth.

The cartridge holder 62 is made up of two halves or a front and rear plate members joined together, and comprises a holding region 62a that extends horizontally, and supporting legs 62b that are disposed at opposite ends of the holding region 62a and extend vertically. The supporting legs 62b are respectively inserted into vertical grooves 61c in the columns 61a of the stand 61 so as to be movable up and down. The supporting leg 62b in the groove 61c is urged upward by an urging member (not shown) incorporated in the stand 61. A plurality of holding holes 62c is provided in the holding region 62a so as to be arranged in a row. The extraction cartridges 11 are inserted into the holding holes 62c from above, and the lower ends of the projections 11d formed on the axially opposite sides of the tubular body 11a of each extraction cartridge 11 (see Figure 11) are engaged with and held by a latching member (not shown) provided in the cartridge holder 62. The latching member is movable out of engagement with the projections 11d to cause all of the extraction cartridges 11 to be dropped at a time for disposal.

Pin-receiving holes 62d, into which a tip 49a of a retaining pin 49 (described later) is received and then the retaining pin 49 itself is pressed down, is formed on either side of the upper surface of the cartridge holder 62 (see Figure 1). When the cartridge holder 62 is in an elevated position as shown in Figure 3, the lower end of the drain portion 11c of the extraction cartridge 11 held in the cartridge holder 62 is positioned above the waste liquid container 12 and collection container 13 held in the container holder 63. Meanwhile, when the cartridge holder 62 is in a lowered position as shown in Figure 4, the drain portion 11c of the extraction cartridge 11 is inserted into the waste liquid container 12 or collection container 13 to a position spaced a predetermined distance from the leading end thereof.

The container holder 63 is provided with a pair of holding zones extending in a lateral direction: one is a row of waste liquid container holding holes 63a for holding a plurality of the waste liquid containers 12 so as to be arranged side by side in a row, and the other is a row of collection container holding holes 63b for holding a plurality of the collection containers 13 so as to be arranged side by side in a row. The waste liquid container holding holes 63a and the collection container holding holes 63b are provided with the same spacing of the holding holes 62c of the cartridge holder 62 such that each waste liquid container 12 or each collection container 13 can be located essentially beneath each of the plurality of extraction cartridges 11 held in place. In order to avoid confusion of the waste liquid container 12 and the collection container 13, it is preferable that the waste liquid container 12 and the collection container 13 differ from one another, for example, in size and/or shape.

The container holder 63 is urged forward by an urging member (not shown) incorporated in the stand 61. When the container holder 63 is moved (forwards or backwards) to replace the containers, an actuating member 31 provided in the holding stage 21 (see Figure 2) is engaged with an engaging hole (not shown) formed in the bottom of the container holder 63 through an opening formed in the bottom plate 61b of the stand 61. As the actuating member 31 is moved with the driving of a container replacement motor 32 (DC motor), the container holder 63 is retracted such that the collection containers 13 are positioned below the cartridge holder 62. When the actuating member 31 is not operational, the container holder 63 is urged by the urging member (not shown) such that the waste liquid containers 12 are positioned below the cartridge holder 62. The operation of the container-replacement motor 32 is controlled on the basis of the detection result by position sensors 33a and 33b.

Each of the waste liquid container holding hole 63a and the collection container holding hole 63b is closed at its bottom end. Therefore, if any liquid accidentally drops with the waste liquid container 12 or the collection container 13 not being mounted in place, the dropped liquid does not go out of container holder 63, whereby the possibility of contamination is prevented.

### <Pressurized Air Supply Mechanism>

The pressurized air supply mechanism 4 comprises a pressing head 40 provided to be vertically movable with respect to the rack 6 of the holding mechanism 3 described above; a plurality of air nozzles 41 (eight nozzles, in Figure 1) that are provided in the pressing head 40 so as to be arranged in a row; an air pump 43 for producing and feeding pressurized air as shown in the air system diagram of Figure 5; a plurality of opening and closing valves 45 which are associated with the air nozzles 41 in one-to-one correspondence for individually stopping or regulating the supply of the pressurized air from the air pump 43; a plurality of separate pressure relief valves 44a, which are associated with the air nozzles 41 in one-to-one correspondence, for individually opening the extraction cartridges 11 to the atmosphere to relieve the internal pressure of each extraction cartridge; a relief valve 44b for opening an air passage situated directly downstream from the air pump 43; a plurality of separate pressure sensors 46a which are respectively provided in the air nozzles 41 and serve to respectively detect the internal pressure of the extraction cartridges 11; and a pressure sensor 46b for detecting a discharge pressure of the air pump 43. The pressurized air supply mechanism 4 serves to feed pressurized air sequentially to the extraction cartridges 11.

The pressing head 40 is supported by guide rods 24 so as to be movable up and down. Each guide rod 24 extends between an intermediate frame 22 and an upper frame 23 of the main body 2. A bore screw 25, which is also provided such that its longitudinal axis extends vertically, is screwed with a ball nut 40a provided in the pressing head 40. As the driving of a lifting and lowering motor 47 (pulse motor) involves the rotation of the bore screw 25 via a timing belt and a pulley, the pressing head 40 is vertically moved under control according to the detection results obtained by photo sensors 48a to 48c. Retaining pins 49 are disposed on either side of the pressing head 40. Each retaining pin 49 is movable up and down under the downward urge of a spring 49b, and a tip 49a of the retaining pin 49 is engages with a pin-receiving hole 62d formed in the upper surface of the cartridge holder 62 and serves to press down the cartridge holder 62 while restricting the position of the holder.

The retaining pin 49 is provided such that the retaining pin 49 holds down the front part of the cartridge holder 62 without interfering the horizontal movement of a washing liquid dispensing nozzle 51w and a recovery liquid dispensing nozzle 51r (described later).

The air nozzles 41 are disposed in the pressing head 40 in a state urged downward to be movable vertically. Under the air nozzles 41, a sheet-like sealing member 42 having through holes 42a corresponding to the air nozzles 41 is provided (see Figure 2). When the pressing head 40 is lowered, the upper opening of each extraction cartridge 11 held in the cartridge holder 62 is pressed by the tip of each air nozzles 41 via the sealing member 42, so that the cartridge 11 is sealed. As a result, it becomes possible to feed pressurized air into the extraction cartridges through the through holes 42a.

The pressure relief valves 44a respectively associated with the air nozzles 41 in one-to-one correspondence are normally in a state in which air passage is open from the opening and closing valves 45 to the air nozzles 41. Pressure relief by the pressure relief valves 44 takes place depending on the determination whether the pressurized state ceases, that is, whether all the liquid within the extraction cartridge 11 is completely drained. At this time, the pressurized air remaining in the extraction cartridges 11 is relieved to the atmosphere, whereby blowing away of the liquid by gushing from the tip of the drain portion 11c is prevented. In the embodiment represented in Figure 5, the pressure relief valve 44a is a three-way solenoid valve, but the pressure relief valve 44a may be a two-way solenoid valve connected on a line branched from the air passage as is the relief valve 44b.

The relief valve 44b (see Figure 5) disposed directly downstream the air pump 43 is normally placed in its closed position and carries out pressure relief to the atmosphere when all of the opening and closing valves 45 are closed. The relief valve 44b further relieves the excessively pressurized air by the continuously operated air pump 43 such that the air of a pressure increased during continuous operation of the air pump is prevented from being suddenly supplied into the extraction cartridges 11.

An air-flow path is provided such that the opening and closing valves 45 (represented as a two-way solenoid valve in Figure 5) are individually opened and the pressurized air from the air pump 43 is individually introduced into the extraction cartridges 11 via the corresponding air nozzles 41.

The pressure sensors 46a are respectively associated with the air nozzles 41 in one-to-one correspondence and serve to individually detect the internal pressure of the extraction cartridges 11. When the pressure sensor 46a detects that the pressure reaches a predetermined pressure specified as an upper limit of the pressurization (for example, 90 kPa), the detection is transferred to a control unit 8 (see Figure 6). In response, the control unit 8 causes the corresponding opening and closing valve 45 to close in order to stop feeding of the pressurized air, determines whether the pressurization state ceases depending on the detection of the reduced pressure associated with the completion of the drainage from the extraction cartridge 11. If determined so, the control unit 8 causes the pressure relief valve 44a to open and controls the system to proceed to the next process.

The pressure sensor 46a serves to detect presence of an extraction cartridge 11 loaded in the cartridge holder 62, presence of a liquid within an extraction cartridge 11, insufficiency of the liquid amount within the extraction cartridge and clogging of the filter, depending on the pressure fluctuations in the extraction cartridge 11. Details will be described later in connection with Figure 9.

Figure 6 shows an air-flow path control system for a single extraction cartridge 11, in which driving control of an air pump 43, actuation of a pressure relief valve 44a and 44b, and individual actuation of an opening and closing valve 45 take place under control of the control unit 8. This control unit 8 controls the pressure to be applied to the extraction cartridge 11, in accordance with a program installed therein, on the basis of a detected pressure signal from the pressure sensors 46a and 46b, an input operation signal from an operation panel 7 disposed at the upper part of the main body 2, and a sample liquid type signal from a data reader 9.

With this configuration, the opening and closing valves 45 are individually opened, thereby introducing pressurized air into the extraction cartridges associated with the valves 45 thus opened. The opening and closing valve 45 in question is closed when the internal pressure detected by the pressure sensor 46a reaches a predetermined pressure specified as an upper limit of the pressurization and then the extraction cartridge 11 is sealed with the inside of the extraction cartridge 11 being pressurized. Subsequently, a pressure is applied to each extraction cartridge 11 to drain the liquid therein through the filter member 11b. Depending on the lowered internal pressure upon completion of the drainage, the pressure sensors 46a respectively check the extraction cartridges 11 associated therewith to detect the cease of the pressurized state, i.e., the time of the completion of the drainage thereof. These detections are used for controlling the pressure relief of the individual extraction cartridges by the pressure relief valves 44a. Further, the discharge flow rate of the air pump 43 is corrected based on the detections from the pressure sensors 46b such that the amount and pressure of the pressurized air supplied into each extraction cartridge 11 reach predetermined values within a predetermined time from the time when the opening and closing valve 45 associated therewith is opened.

Further, depending on an input operation signal from the operation panel 7, a sample liquid type signal from the data reader 9 and the like, the control unit 8 changes control parameters including the aforementioned predetermined pressure specified as an upper limit of the pressurization such that operations are performed with the processing procedure (protocol) and set value most appropriate for the type of the sample liquid S. Specifically, the control unit 8 addresses the change of the type of the sample liquid S to be processed by changing the pressurization control method, the amount of the liquid to be processed, and/or the number of washing times, or by additively dispensing a reagent. For example, a protocol, set value and the like which have been programmed beforehand may be selected and controlled, for example, by keying in the sample type and the like via a keyboard of the operation panel 7. In the case where different reagents are used for each sample type to be processed, a protocol, a set value and the like which have been programmed beforehand may be selected and controlled, for example, by reading the barcodes or the information on the IC chip attached to the reagents with the data reader 9. Similarly, a protocol and/or a set value may be changed by reading a data storage element such as CF card (memory card), on which the information representing the reagent is recorded, by the data reader 9.

In the pressurization, pressurized air may be introduced into a plurality of the extraction cartridges 11 simultaneously or sequentially one-by-one. In order to introduce the pressurized air in the extraction cartridges 11 sequentially one-by-one, one of the opening and closing valve 45, all of which are in their closed positions, is opened to introduce the pressurized air into the extraction cartridge 11 associated with the valve 45 in question. Then, this opening and closing valve 45 is opened according to the detection from the corresponding pressure sensor 46a. Subsequently, the next one of the opening and closing valves 45 is subjected to the procedure of opening the valve 45, introducing the extraction cartridges 11 associated with this valve 45, and closing this valve 45 according to the detection from the corresponding pressure sensor 46a. These procedures are repeated for each of the remaining valves 45. Thus, the pressurized air is introduced into the plurality of extraction cartridges.

Meanwhile, in order to simultaneously introduce the pressurized air into a plurality of extraction cartridges 11, the corresponding number of opening and closing valves 45, all of which are in their closed positions and respectively associated with the plurality of extraction cartridges 11, are opened to introduce the pressurized air into the plurality of extraction cartridges 11. The plurality of the opening and closing valves 45 are individually closed according to the detections from the corresponding pressure sensors 46a. In the case where the pressurized air is simultaneously introduced into the plurality of extraction cartridges, the discharge rate of the air pump 43 is controlled depending on the number of the opening and closing valves 45 to be opened, such that the rate of pressure rise remains constant. Such a discharge rate control for this air pump 43 is performed, for example, based on the pulse width modulation (PWM). Specifically, a voltage to be applied to the air pump 43 is varied by appropriately controlling the time ratio (on-off duty cycle control) depending on the number of the extraction cartridges 11 such that the rotational frequency of the air pump 43 increases with increase of the applied pressure, and a constant rate of pressure rise of the pressurized air to be introduced in the extraction cartridges 11 is made constant, whereby the pressure of the air sealed when the opening and closing valves 45 are closed in response to the detection of the predetermined pressure specified as an upper limit of the pressurization the by the pressure sensor 46a is kept constant. The discharge rate control described above can also be performed in a corresponding manner. While the operations of detecting completion of the drainage under the controlled pressure by the control unit 8 will be described later in connection with the flowcharts shown in Figures 7 and 8, the control characteristics when a sample contained in a sample liquid with an unknown viscosity is pressurized (Figure 7) and the control characteristics when a washing liquid and a recovery liquid are pressurized (Figure 8) are different from one another. Specifically, when the sample is pressurized, the completion of the drainage is detected based on both the acceleration of decrease in pressure and the amount of decreased pressure. Meanwhile, when the washing liquid and the recovery liquid are pressurized, the completion of the drainage is detected only based on amount of decreased pressure.

### <Dispensing Mechanism>

The dispensing mechanism 5 includes a washing liquid dispensing nozzle 51w mounted on a nozzle slide 50 which is horizontally movable above the rack 6; a recovery liquid dispensing nozzle 51r, a washing liquid supply pump 52w for feeding a washing liquid W held within a washing liquid bottle 56w to the washing liquid dispensing nozzle 51w, a recovery liquid supply pump 52r for feeding a recovery liquid R held in a recovery liquid bottle 56r to the recovery liquid dispensing nozzle 51r, a waste liquid bottle 57 placed on the holding stage 21, and so on.
The nozzle slide 50 is supported on a guide rail 27 extending horizontally along a vertical wall 26 of the main body 2 and thus laterally movable. The nozzle slide 50 is controlled by a nozzle moving motor (i.e., pulse motor, not shown) such that the nozzle slide 50 is sequentially brought to and stopped at a plurality of positions each correspondingly situated above the respective cartridges 11 and returned as required to a position above the waste liquid bottle 57. The distal end of the washing liquid dispensing nozzle 51w and the distal end of the recovery liquid dispensing nozzle 51r are both bent downward. The washing liquid dispensing nozzle 51w is connected to the washing liquid supply pump 52w via a selector valve 55w, the washing liquid supply pump 52w is connected to the washing liquid bottle 56w via a selector valve 55w, the recovery liquid dispensing nozzle 51r is connected to the recovery liquid supply pump 52r via a selector valve 55r, and the recovery liquid supply pump 52r is connected to recovery liquid bottle 56r via a selector valve 55r. The washing liquid bottle 56w and the recovery liquid bottle 56r are both attached on a side wall of the system main body. The washing liquid supply pump 52w and the recovery liquid supply pump 52r are both composed of a syringe pump. A piston member of washing liquid supply pump 52w is controlled by a pump motor 53w (pulse motor) to dispense a predetermined amount of a washing liquid W according to a position detection from a sensor 54w, while a piston member of the recovery liquid supply pump 52r is controlled by a pump motor 53r (pulse motor) to dispense a predetermined amount of a recovery liquid R according to a position detection from a sensor 54r. More particularly, in order to dispense the washing liquid W, the selector valve 55w is actuated to select the washing liquid bottle 56w, the pump motor 53w is driven to retract the piston member of the washing liquid supply pump 52w to suction the washing liquid W into the washing liquid supply pump 52w. Subsequently, the selector valve 55w is actuated to select the washing liquid dispensing nozzle 51w, the pump motor 53w is driven to push the piston member of the washing liquid supply pump 52w to cause the washing liquid to be drained through the washing liquid dispensing nozzle 51w into the waste liquid bottle 57 until the air within the passage is discharged, and the washing liquid supply pump 52w is stopped. After that, the washing liquid dispensing nozzle 51w is moved above the extraction cartridge 11, and the washing liquid supply pump 52w is controlled such that a predetermined amount of the washing liquid W is dispensed into the extraction cartridge 11. Meanwhile, in order to dispense the recovery liquid R, the selector valve 55r is actuated to select the recovery liquid bottle 56r, the pump motor 53r is driven to retract the piston member of the recovery liquid supply pump 52r to suction the recovery liquid R into the recovery liquid supply pump 52r. Subsequently, the selector valve 55r is actuated to select the recovery liquid dispensing nozzle 51r, the pump motor 53r is driven to push the piston member of the recovery liquid supply pump 52r to cause the recovery liquid to be drained through the recovery liquid dispensing nozzle 51r into the waste liquid bottle 57 until the air within the passage is discharged, and the recovery liquid supply pump 52r is stopped. After that, the recovery liquid dispensing nozzle 51r is moved above the extraction cartridge 11, and the recovery liquid supply pump 52r is controlled such that a predetermined amount of the recovery liquid R is dispensed into the extraction cartridge 11.

The foregoing control unit 8 (see Figure 6) serves to control not only the pressurization, but also the operations of the foregoing mechanisms 3 to 5. Specifically, the control unit 8 controls a series of processing steps for automatic extraction on the basis of an input operation through the operation panel 7 disposed at the upper part of the main body 2 in accordance with a program installed therein.

In the following, the extracting operation of the foregoing extraction system 1 will be described in detail. First, an extraction cartridge 11 is loaded in the cartridge holder of the rack 6 of the loading mechanism 3, the waste liquid container 12 and the collection container 13 are respectively loaded in the container holder 63, and the rack 6 is placed on the holding stage 21 of the main body. Then, one or more solubilized sample liquids S are sequentially injected into the extraction cartridges 11, for example, by a pipette. The sample liquid S may be injected into the extraction cartridges 11 before or after being loaded in the rack 6, prior to the rack 6 being mounted on the system 1.

Then, when the system is actuated according to an operation of the operation panel 7 by an operator, a lifting and lowering motor 47 of the pressurized air supply mechanism 4 is driven so that the pressing head 40 is moved downward. Accordingly, the tip 49a of the retaining pin 49 is engaged with the pin-receiving hole 62d of cartridge holder 62, presses the cartridge holder 62, and hence moves the cartridge holder 62 downwards while restricting the position of the cartridge holder 62. At this time, the drain portion 11c located at the bottom of the extraction cartridge 11 is inserted into the waste liquid container 12 to a position spaced a predetermined distance from the leading end of the cartridge as shown in Figure 4, thereby preventing the drained liquid being leaked outside by blowing away or the like. Further, the pressing head 40 is moved downward, whereby the lower end of each air nozzle 41 is brought in sealing abutment against the upper opening of each extraction cartridge 11 via the sealing member 42. Since the retaining pin 49 restricts the position of the cartridge holder 62, each air nozzle 41 can be brought in accurate sealing abutment against the each extraction cartridge 11, whereby a reliable sealing is ensured.

Thereafter, pressurized air is supplied to the extraction cartridges 11. One exemplary routine of controlling the pressurization of a sample will be described in connection with the flowchart shown in Figure 7. This flowchart shows a control routine for a single extraction cartridge 11. After application of the pressure starts, in step S1, the opening and closing valve 45 is opened and the air pump 43 is driven. Pressurized air from the air pump 43 is supplied to the extraction cartridge 11 through an air nozzle 41 associated with the extraction cartridge 11, and a pressure sensor 46a, also associated with the extraction cartridge 11, detects the pressure in the cartridge 11, and whether the detected pressure reaches the predetermined upper limit pressure is determined (step S2).

When the supplied pressure reaches the upper limit and the answer in step S2 becomes YES, the opening and closing valve 45 is closed (step S3) whereby the extraction cartridge 11 is sealed with the inside thereof being pressurized. In the extraction cartridge 11 in which the pressurized air has been introduced, by the action of the pressure, the sample liquid S is passed through the filter member 11b while a nucleic acid is adsorbed to and held on the filter member 11. Other liquid components, which are passed through the filter, are then drained from the lower end of the drain portion 11c of the cartridge 11 into the waste liquid container 12.

Subsequently, in steps S4 and S5, whether the drainage of the liquid has been completed is determined. In particular, step S4 determines whether the acceleration of decrease in pressure reaches or exceeds a predetermined value "x" kPa/sec². This step is that which accurately detects pressure fluctuations upon completion of the drainage from the cartridge regardless of the varying components such as a viscosity of the sample liquid S, a pressurization rate, and the like, by differentiating the pressure fluctuation associated with the drainage twice, based on the fact that internal pressure of the cartridge abruptly reduces after the liquid therein is substantially drained. If the answer in step S4 is NO, i.e., the determination by step S4 is unavailable, step S5 determines whether the pressure reduction exceeds a predetermined relief pressure (threshold value). Specifically, in step S5, the moment that the pressure difference between the upper limit pressure when the opening and closing valve 45 is closed and the decreased pressure within the cartridge becomes larger than the value thus predetermined is determined as the completion of the drainage. If it is determined in Step S4 or S5 that the drainage of the liquid from the extraction cartridge 11 is completed (i.e., the answer either in step S4 or S5 is YES), the pressure relief valve 44a is relieved to the atmosphere in step S6, and the pressurization to the extraction cartridge 11 terminates.

Pressurized air is similarly supplied to each extraction cartridge 11 and the foregoing procedure is repeated for each cartridge until all of the extraction cartridges 11 are pressurized. When completion of the drainage is detected for all of the extraction cartridges by the respective pressure sensors 46a, the pressurization process itself terminates and the pressing head 40 is moved upward.

As described above, when the pressure sensor 46a detects the cease of the pressurization where the internal pressure abruptly drops upon completion of the drainage after a sample liquid S has all passed through the filter member 11b, the associated pressure relief valve 44a is opened to relieve the pressurized air remained in the extraction cartridge 11 to the atmosphere, thereby preventing the pressurized air from being violently expelled together with the sample liquid from the rain portion 11c of the cartridge 11.

Hereinafter, a description will given similarly with reference to the flowchart shown in Figure 8 on an exemplary manner of pressure control in the course of the pressurizing step after injection of a washing liquid W or a recovery liquid R for washing or collecting the nucleic acid, after the nucleic acid is separated by pressurizing the sample liquid. After pressurization is started, the opening and closing valve 45 is opened in step S11, whereby pressurization air is supplied to the extraction cartridges 11 through the air nozzles 41. Whether the pressure detected by the pressure sensor 46a reaches a predetermined upper limit pressure is determined (step S12). When the supplied pressure reaches the upper limit pressure and the answer in step S12 becomes YES, the opening and closing valve 45 is closed (step S13). After the extraction cartridge 11 has been supplied with the pressurized air and sealed, the washing liquid W or the recovery liquid R is drained through the filter member 11b by the action of the pressure.

Then step S14 determines whether the drainage is complete. The viscosity of the washing liquid W or recovery liquid R is low and known. At this time, there is no need to determine the acceleration of decrease in pressure. More specifically, step S14 is similar to step S5 shown in the flowchart in Figure 7, which determines whether the internal pressure reduction exceeds a relief pressure (threshold value). If the answer in step S14 is YES, i.e., if step S14 determines that the drainage of the liquid from the extraction cartridge 11 is completed, the pressure relief valve 44a is operated to relieve pressure to the atmosphere in step S15, and the pressurization to the extraction cartridge 11 is terminated.

Figure 9 shows the pressure fluctuations inside an extraction cartridge 11 associated with the introduction of pressurized air into the extraction cartridge 11: curve A represents the characteristics of the internal pressure fluctuation under a normal working condition; and curve B represents the differential waveform representing the rate of change of pressure. First, the pressure linearly rises from time point "0" (zero) when the opening and closing valve 45 is opened to start introduction of pressurized air. Then, at time point "a" when the pressure reaches a predetermined upper pressure limit (for example, 90 kPa), the opening and closing valve 45 is closed and the internal space of the extraction cartridge is sealed with the inside thereof being pressurized. This pressure acts to pass a liquid within the cartridge through the filter member 11b and decreases with gradual decrease of the liquid inside the cartridge. Subsequently, as the total amount of the liquid is passed through the filter member and thus drainage of the liquid is completed at time point "b", air resistance at the filter member 11b is reduced and the pressure within the cartridge abruptly decreases. The differential waveform curve B exhibits a remarkable pressure fluctuation. Even if the pressure fluctuation does not remarkably appear in the differential waveform curve B, the pressure fluctuation can be made remarkable by differentiating the pressure fluctuation twice. Thus, cease of the pressurization is determined on the basis that the rate of change of pressure reaches or exceeds a predetermined value "x" kPa/sec². Alternatively, the aforementioned determination regarding the cease of the pressurization is performed on the basis that the pressure reduction associated with the drainage from the pressure at point "a" exceeds a predetermined relief pressure (threshold value).

When the sample liquid S is pressurized as described above, the pressure fluctuation differs depending on its viscosity and the like. In the case of a liquid having lower viscosity, the degree of the pressure reduction from point "a" to "b" increases and the time to "b" becomes shorter. When the viscosity of the liquid is relatively high and minor clogging of the filter member 11b occurs, the degree of the pressure reduction from point "a" to "b" decreases and the time to "b" becomes longer.

Further, based on the internal pressure fluctuation characteristics detected by the pressure sensor 46a, detection of a deficient pressurization state, for example, detection of a presence of an extraction cartridge 11 loaded in the cartridge holder 62, detection of the presence of a liquid within an extraction cartridge 11, detection of insufficient sealing, detection of insufficiency of the liquid amount within the extraction cartridge, or detection of clogging of the filter is performed.

First, the deficient pressurization state is detected when a pressure detected by the pressure sensor 46a does not reach a pressure specified for determining the deficient pressurization, for example, as low as 10 kPa. This represents a faulty state where air resistance is low, that is, an extraction cartridge 11 has not been mounted in place, a sample liquid has not been injected, or the sealing between the air nozzle 41 and the extraction cartridge 11 is insufficient.

When the amount of a sample liquid S injected to an extraction cartridge 11 is not the defined amount but a small amount, the initial pressure rises so as to exceed the pressure specified for determining the deficient pressurization mentioned above, but does not rise to the pressure specified as the upper limit for pressurization where the opening and closing valve 45 is closed, and thus the drainage of the liquids is completed before the pressure specified as the upper limit for pressurization is reached, and the pressure abruptly decreases. Such a case is determined as that in which the liquid amount is insufficient. Further, the case where the detected pressure gradually decreases with the drainage of the liquid, the pressure reduction is small, and after a predetermined time has elapsed, the cease of pressurization upon completion of the drainage cannot be determined and the pressure does not decrease to a value lower than the pressure specified for determining the cease of the pressurization is determined as that in which clogging of a filter has occurred. Clogging of the filter may be detected by the fact that the state where the decrease of pressure does not reach or exceed a predetermined value is maintained for a time over a predetermined time.

The insufficient pressurization detection when the pressure rise is insufficient during pressurization, the detection of the cease of the pressurization associated with the completion of the drainage, and the detection of the clogging of the filter are similarly carried out during the washing and collection processes described later.

Then, control goes to the washing process, during which raising of the pressing head 40 after the pressurized air is supplied should be carried out with the state shown in Figure 4 being maintained, where the air nozzle 41 has been moved away from the extraction cartridge 11 and brought upward to a position where the movement of the nozzle slide 50 is allowed, and the retaining pin 49 is pressing the cartridge holder 62 such that the lower end of the extraction cartridge 11 is inserted within the waste liquid container 12. Then, the nozzle slide 50 is moved to bring the washing liquid dispensing nozzle 51w to the first extraction cartridge 11, where a predetermined amount of a washing liquid W is dispensed in the first extraction cartridge 11, and then the nozzle slide 50 is brought to the next extraction cartridge 11 for dispensing therein the washing liquid W, and so on. After completion of the dispensing of the washing liquid W to all of the extraction cartridges 11, the pressing head 40 is moved downward, whereby the lower end of each air nozzle 41 is brought in sealing abutment against the upper opening of each extraction cartridge 11 via the sealing member 42. Subsequently, the opening and closing valves are sequentially opened to respectively supply pressurized air into the extraction cartridges 11 in a similar manner as described above. The washing liquid W under pressure passes through the filter member 11b and serves to wash out the impurities other than the nucleic acid adsorbed on the filter member 11b. The washing liquid W is drained from the extraction cartridge 11. The pressure relief valve 44a is opened as described above upon completion of the drainage. When the washing liquid W in all of the extraction cartridges 11 has all passed through the filter members 11b and drained, the pressing head 40 is moved upward to its initial position. When the washing process is to be performed more than once, the foregoing process is repeated.

The pressurized air used for the washing process and a collection process (described later) may be supplied simultaneously to a plurality of extraction cartridges 11. Specifically, all the extraction cartridges 11 under normal operating conditions, i.e. the extraction cartridges 11 except those having a portion determined as under a deficient pressurized state or a clogged filter portion are simultaneously opened, pressurized air is introduced therein by the air pump 43, which is variably driven based on the PWM control, and these operating and closing valves 45 are individually closed according to the detections of the upper pressurization limits from the pressure sensor 46a.

Then, control goes to the extraction process. First, an upward movement of the pressing head 50 after the washing process lifts the retaining pin 49, and in turn lifts the cartridge holder 62 of the rack. After the drain portion 11c located at the lower end of the extraction cartridge 11 moved to a position above the waste liquid container 12, the container is replaced with another one by operating the actuating member 31 of the loading mechanism 3 such that the container holder 63 is retracted, and by positioning the collection container 13 under the extraction cartridge 11.

Subsequently, the pressing head 40 is moved downward so that the tip of the retaining pin 49 is engaged with the pin-receiving hole 62d of the cartridge holder 62 and presses the cartridge holder 62. Then, the nozzle slide 50 is moved to bring the recovery liquid dispensing nozzle 51r to the first extraction cartridge 11 where a predetermined amount of a recovery liquid R is dispensed in the first extraction cartridge 11, and then the nozzle slide 50 is brought to the next extraction cartridge 11 for dispensing therein the recovery liquid R, and so on. After completion of the dispensing of the recovery liquid R to all of the extraction cartridges 11, the pressing head 40 is moved downward as described above, whereby the lower end of each air nozzle 41 is brought in sealing abutment against the upper opening of each extraction cartridge 11 via the sealing member 42. Subsequently, the opening and closing valves are sequentially opened to respectively supply pressurized air into the extraction cartridges 11. The recovery liquid R under pressure passes through the filter member 11b, serves to desorb the nucleic acid adsorbed on the filter member 11b, and is drained from the extraction cartridge 11. Then, the recovery liquid R is drained together with the nucleic acid into the collection container 13 from the drain port 11c at the bottom end of the cartridge, and the pressure relief valve 44a is opened as described above upon completion of the drainage. After the recovery liquids R within all of the extraction cartridges 11 are all drained into the collection container 13, the pressing head 50 is moved upward, and thus the sequence of operations is terminated.

After completion of such an extracting operation, the rack 6 is dismounted from the holding stage 21, the extraction cartridge 11 and the waste liquid container 12 are respectively removed from the cartridge holder 62 and the container holder 63 and discarded. Meanwhile the recovery container 13 is removed from the container holder 63, capped as required, and subjected to the next nucleic acid analysis or the like.

In the foregoing description of the embodiment, the washing process by the use of the washing liquid W is employed. However, depending on the filtering characteristics of the filter member 11b, such a washing process is not necessarily required.

Although the foregoing embodiment was described in relation to a nucleic acid extraction system, the present invention is not limited thereto. This invention is also applicable to an extraction system in which various specific materials are brought into contact with a filter member. Further, such specific materials need not necessarily be collected by the use of a recovery liquid. The specific material may be analyzed with the specific material being in contact with the filter member or may be analyzed by adding a process liquid to the specific material and examining the resultant color.

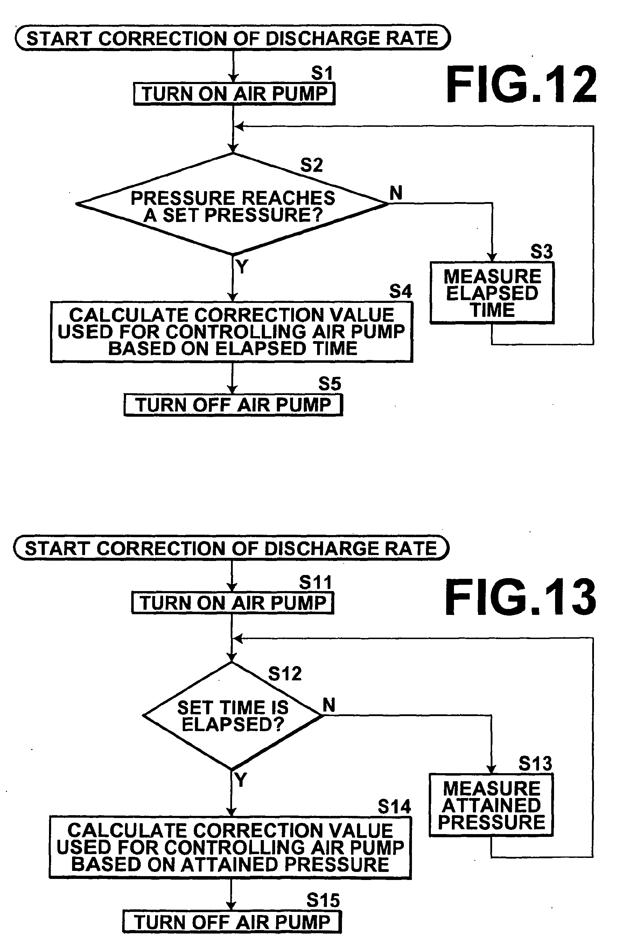

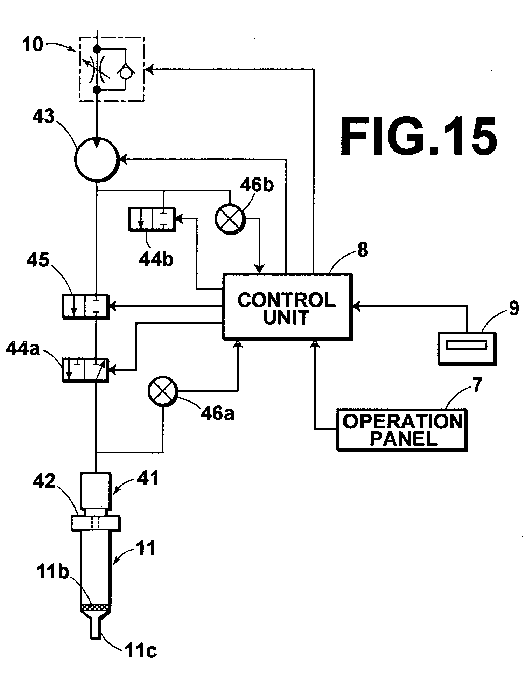

Hereinafter, an extraction system, as an embodiment of the third and fourth extraction systems of the present invention will be described in detail with reference to the drawings. Figure 12 to Figure 14 are flowchart diagrams for illustrating an exemplary routine of controlling pressurization, and Figure 15 shows a control system of a pressurized air supply mechanism of the fourth extraction system of the present invention. Since Figures 1 to 6, Figure 9 and Figure 10 can also be used to illustrate the third and fourth extraction systems, new drawings are not prepared. Specifically, Figures 1 to 6, Figure 9 and Figure 10 also illustrate the third and fourth extraction system of the present invention. Figure 1 is a perspective view of a nucleic acid extraction system according to one embodiment of the invention; Figure 2 is a schematic block diagram of the nucleic acid extraction system; Figure 3 is a perspective view of a rack loaded in a holding mechanism of the nucleic acid extraction system; Figure 4 is a perspective view showing the rack in operation; Figure 5 is a diagram illustrating an air system of pressurized air supply mechanism of the nucleic acid extraction system; Figure 6 is a diagram illustrating a control system of the pressurized air supply mechanism; Figure 9 is a graphical representation showing the variations in the internal pressure of an extraction cartridge; Figures 10A to 10G show processing steps for an extracting operation; and Figure 11 is a perspective view of the extraction cartridge.

Before addressing the features of an exemplary nucleic acid extraction system 1 according to one embodiment of the invention, which is provided as an representative example of the third and fourth extraction systems of the present invention, it should be noted that the nucleic acid extraction system 1 is that which extracts a nucleic acid as a specific material contained in a sample liquid by use of an extraction cartridge 11 (a filter cartridge) shown in Figure 11.

This extraction cartridge 11 comprises a tubular body 11a having an open top. A filter member 11b is retained on the inner bottom of the tubular body 11a. A portion of the tubular body 11a extending below the filter member 11b is shaped in the form of a funnel, and a thin nozzle-like drain portion 11c, centrally disposed on the underside of the tubular body 11a, projects downward over a predetermined length. Projections 11d are formed on the axially opposite sides of the tubular body 11 a such that each projection 11d projects radially outward and extends longitudinally. A sample liquid, a washing liquid and a recovery liquid (each of which will be described later) are individually dispensed through the upper opening of the extraction cartridge 11 and then pressurized air is introduced through the upper opening, whereby each liquid flows down through the filter member 11b and is discharged from the drain portion 11c into a waste liquid container 12 or a collection container 13 (each of which will be described later). In this particular embodiment, the tubular body 11a is composed of an upper part and a lower part which are engaged with each other.

In principle, the nucleic acid extraction system 1 extracts and purifies a nucleic acid according to an extraction procedure comprising the steps as shown in Figure 10A to Figure 10G. Hereinafter, the step shown in Figure 10A is referred to as step (a), the step shown in Figure 10B is referred to as step (b), and so on. First, in step (a), a sample liquid S containing the nucleic acid which has been subjected to solubilization are injected into the extraction cartridge 11 positioned above the waste liquid container 12. Then, in step (b), pressurized air is introduced into the extraction cartridge 11 so that the sample liquid S is passed through the cartridge via the filter member 11b, the nucleic acid is adsorbed to the filter member 11b, and the liquid component is drained through the cartridge 11 into the waste liquid container 12.

Next, in step (c), a washing liquid W is automatically dispensed in the extraction cartridge 11. In step (d), pressurized air is introduced in the extraction cartridge 11 to apply pressure thereto, so that the impurities other than the nucleic acid are removed by washing while the nucleic acid remains on the filter member 11b. The washing liquid W through the cartridge is drained into the waste liquid container 12. Steps (c) and (d) may be repeated.

After that, in step (e), the waste liquid container 12 positioned below the extraction cartridge 11 is replaced with the collection container 13, and in step (f), a recovery liquid R is automatically dispensed in the extraction cartridge 11, pressurized air is introduced into the extraction cartridge 11 to apply pressure thereto so that the biding force between the nucleic acid and the filter member 11b is weakened to separate the adsorbed nucleic acid therefrom, and the recovery liquid R, in which the nucleic acid is contained, is drained from the extraction cartridge 11 and collected into the collection container 13.

The filter member 11b in the extraction cartridge 11 described above is basically porous, to enable nucleic acid to pass therethrough, and includes a surface having a property of adsorbing a nucleic acid contained in a sample liquid with a chemical binding force. Thus, the filter member 11b is designed such that the adsorption of the nucleic acid is maintained when the filter member 11b undergoes a washing process using a washing liquid, while the adsorbing force is weakened and the nucleic acid is separated from the filter member 11b when the filter member 11b undergoes a collection process using a recovery liquid. As described in detail in U.S. Patent Application Publication No. 20030170664 regarding a method of isolating and purifying nucleic acid, the foregoing filter member 11b, for example, may be composed of organic high polymers having a hydroxyl group on a surface thereof. Surface-saponified cellulose acetate is preferred as the organic high polymer having a hydroxyl group on its surface. Any cellulose acetate such as cellulose monoacetate, cellulose diacetate, and cellulose triacetate may be used, but in particular, cellulose triacetate is preferable. The surface that comes in contact with saponification treatment solution (e.g., NaOH) is saponified, while the structure remains cellulose acetate. This allows the amount of hydroxyl group (density) on the surface to be controlled according to the degree of surface saponification (surface saponification degree). The adsorbing effect of nucleic acids increases as a larger number of hydroxyl groups are present. For example, in the case of cellulose acetate such as cellulose triacetate, the surface-saponification ratio is preferably 5% or more, more preferably 10% or more. Cellulose acetate is preferably provided in the form of a porous membrane.

The "sample liquid S containing nucleic acids" is a solution which is prepared by adding an aqueous organic solvent to a solution obtained by subjecting a sample containing a cell or a virus to the solubilization. For example, in the field of diagnostics, the sample liquids S include: body fluids collected as samples, such as whole blood, plasma, serum, urine, stool, sperm, and saliva; plants (or a part thereof); animals (or a part thereof); and solutions prepared from biological materials such as lysates and homogenates of the above samples. The "solubilization" is a treatment for treating a sample with an aqueous solution containing a reagent capable of dissolving cell membranes and solubilizing nuclear membranes (for example, a solution comprising a guanidine salt, a surfactant and a protease). For example, when a sample is whole blood, erythrocytes and various proteins are broken and depolymerized in order to prevent their non-specific adsorption to the filter member 11b and clogging thereof, and lysis of leukocytes and nuclear membranes is carried out to solubilize the nucleic acids which are to be extracted. The "water-soluble organic solvents" to be used herein include ethanol, isopropanol, and propanol. Among these, ethanol is preferable. The concentration of the water-soluble organic solvent is preferably 5% by weight to 90% by weight, more preferably 20% by weight to 60% by weight. It is particularly preferable that ethanol is added so as to have as high a concentration as possible, but to such an extent that aggregation does not occur.

The "washing liquid W" serves to wash out impurities which were present in the sample liquid and were adsorbed onto the filter member 11b with the nucleic acids. This liquid has a composition by which only impurities are desorbed from the filter member 11b with the nucleic acids remaining adsorbed. The washing liquid W is a solution containing a base agent and a buffer agent, and optionally a surfactant. The base agents include aqueous solutions having approximately 10 to 100% by weight (preferably approximately 20 to 100% by weight, more preferably approximately 40 to 80% by weight) of methanol, ethanol, isopropanol, n-isopropanol, butanol, actone, and the like.

The "recovery liquid R" has preferably a low salt concentration, and more preferably a solution having a salt concentration of 0.5 M or lower is used. Examples of the liquid to be used include purified distilled water and TE buffers.

As shown in Figures 1 and 2, the nucleic acid extraction system 1 comprises: a main body 2 of the nucleic acid extraction system 1; a holding mechanism 3 for holding a plurality of extraction cartridges 11, a plurality of waste liquid containers 12, and a plurality of collection containers 13 in the main body 2; a pressurized-air supply mechanism 4 for introducing pressurized air into the extraction cartridges 11; and a dispensing mechanism 4 for dispensing a washing liquid W and a recovery liquid R into the extraction cartridges 11; etc. In the following, each mechanism 3 to 5 will be specifically described.

### <Holding Mechanism>

The holding mechanism 3 comprises a holding stage 21 disposed at a front lower part of the main body 2. A rack 6, in which the extraction cartridges 11, the waste liquid containers 12, and the recovery containers 13 are held, is mounted on the holding stage 21. As also shown in Figure 3, the rack 6 comprises a stand 61, a cartridge holder 62, and a container holder 63.

The stand 61 comprises opposite columns 61a which cooperate to support the cartridge holder 62 in a manner to be movable up and down, and a bottom plate 61b that is disposed between the columns 61a and supports thereon the container holder 63 in a manner so as to be movable back and forth.

The cartridge holder 62 is made up of two halves or a front and rear plate members joined together, and comprises a holding region 62a that extends horizontally, and supporting legs 62b that are disposed at opposite ends of the holding region 62a and extend vertically. The supporting legs 62b are respectively inserted into vertical grooves 61c in the stand 61 so as to be movable up and down. The supporting leg 62b in the groove 61c is urged upward by an urging member (not shown) incorporated in the stand 61. A plurality of holding holes 62c is provided in the holding region 62a so as to be arranged in a row. The extraction cartridges 11 are inserted into the holding holes 62c from above, and the lower ends of the projections 11d formed on the axially opposite sides of the tubular body 11a of each extraction cartridge 11 (see Figure 11) are engaged with and held by a latching member (not shown) provided in the cartridge holder 62. The latching member is movable out of engagement with the projections 11d to cause all of the extraction cartridges 11 to be dropped at a time for disposal.

Pin-receiving holes 62d, into which a tip 49a of a retaining pin 49 (described later) is received and then the retaining pin 49 itself is pressed down, is formed on either side of the upper surface of the cartridge holder 62 (see Figure 1). When the cartridge holder 62 is in an elevated position as shown in Figure 3, the lower end of the drain portion 11c of the extraction cartridge 11 held in the cartridge holder 62 is positioned above the waste liquid container 12 and collection container 13 held in the container holder 63. Meanwhile, when the cartridge holder 62 is in a lowered position as shown in Figure 4, the drain portion 11c of the extraction cartridge 11 is inserted into the waste liquid container 12 or collection container 13 to a position spaced a predetermined distance from the leading end thereof.

The container holder 63 is provided with a pair of holding zones extending in a lateral direction: one is a row of waste liquid container holding holes 63a for holding a plurality of the waste liquid containers 12 so as to be arranged side by side in a row, and the other is a row of collection container holding holes 63b for holding a plurality of the collection containers 13 so as to be arranged side by side in a row. The waste liquid container holding holes 63a and the collection container holding holes 63b are provided with the same spacing of the holding holes 62c of the cartridge holder 62 such that each waste liquid container 12 or each collection container 13 can be located essentially beneath each of the plurality of extraction cartridges 11 held in place. In order to avoid confusion of the waste liquid container 12 and the collection container 13, it is preferable that the waste liquid container 12 and the collection container 13 differ from one another, for example, in size and/or shape.

The container holder 63 is urged forward by an urging member (not shown) incorporated in the stand 61. When the container holder 63 is moved (forwards or backwards) to replace the containers, an actuating member 31 provided in the holding stage 21 (see Figure 2) is engaged with an engaging hole (not shown) formed in the bottom of the container holder 63 through an opening formed in the bottom plate 61b of the stand 61. As the actuating member 31 is moved with the driving of a container replacement motor 32 (DC motor), the container holder 63 is retracted such that the collection containers 13 are positioned below the cartridge holder 62. When the actuating member 31 is not operational, the container holder 63 is urged by the urging member (not shown) such that the waste liquid containers 12 are positioned below the cartridge holder 62. The operation of the container-replacement motor 32 is controlled on the basis of the detection result by position sensors 33a and 33b.

Each of the waste liquid container holding hole 63a and the collection container holding hole 63b is closed at its bottom end. Therefore, if any liquid accidentally drops with the waste liquid container 12 or the collection container 13 not being mounted in place, the dropped liquid does not go out of container holder 63, whereby the possibility of contamination is prevented.

### <Pressurized Air Supply Mechanism>

The pressurized air supply mechanism 4 comprises a pressing head 40 provided to be vertically movable with respect to the rack 6 of the holding mechanism 3 described above; a plurality of air nozzles 41 (eight nozzles, in Figure 1) that are provided in the pressing head 40 so as to be arranged in a row; an air pump 43 for producing and feeding pressurized air as shown in the air system diagram of Figure 5; a plurality of opening and closing valves 45, which are associated with the air nozzles 41 in one-to-one correspondence, for individually stopping or regulating the supply of the pressurized air from the air pump 43; a plurality of separate pressure relief valves 44a which are associated with the air nozzles 41 in one-to-one correspondence for individually opening the extraction cartridges 11 to the atmosphere to release the internal pressure of each extraction cartridge; a relief valve 44b for opening an air path situated directly downstream from the air pump 43; a plurality of separate pressure sensors 46a which are respectively provided in the air nozzles 41 and serve to respectively detect the internal pressure of the extraction cartridges 11; and a pressure sensor 46b for detecting a discharge pressure of the air pump 43. The pressurized air supply mechanism 4 serves to feed pressurized air sequentially to the extraction cartridges 11.

The pressing head 40 is supported by guide rods 24 so as to be movable up and down. Each guide rod 24 extends between an intermediate frame 22 and an upper frame 23 of the main body 2. A bore screw 25, which is also provided such that its longitudinal axis extends vertically, is screwed with a ball nut 40a provided in the pressing head 40. As the driving of a lifting and lowering motor 47 (pulse motor) involves the rotation of the bore screw 25 via a timing belt and a pulley, the pressing head 40 is vertically moved under control according to the detection results obtained by photo sensors 48a to 48c. Retaining pins 49 are disposed on either side of the pressing head 40. Each retaining pin 49 is movable up and down under the downward urge of a spring 49b, and a tip 49a of the retaining pin 49 is engages with a pin-receiving hole 62d formed in the upper surface of the cartridge holder 62 and serves to press down the cartridge holder 62 while restricting the position of the holder.

The retaining pin 49 is provided such that the retaining pin 49 holds down the front part of the cartridge holder 62 without interfering the horizontal movement of a washing liquid dispensing nozzle 51w and a recovery liquid dispensing nozzle 51r (described later).

The air nozzles 41 are disposed in the pressing head 40 in a state urged downward to be movable vertically. Under the air nozzles 41, a sheet-like sealing member 42 having through holes 42a corresponding to the air nozzles 41 is provided (see Figure 2). When the pressing head 40 is lowered, the upper opening of each extraction cartridge 11 held in the cartridge holder 62 is pressed by the tip of each air nozzles 41 via the sealing member 42, so that the cartridge 11 is sealed. As a result, it becomes possible to feed pressurized air into the extraction cartridges through the through holes 42a.

The pressure relief valves 44a respectively associated with the air nozzles 41 in one-to-one correspondence are normally in a state in which air passage is open from the opening and closing valves 45 to the air nozzles 41. Pressure relief by the pressure relief valves 44 takes place depending on the determination whether the pressurized state ceases, that is, whether all the liquid within the extraction cartridge 11 is completely drained. At this time, the pressurized air remaining in the extraction cartridges 11 is relieved to the atmosphere, whereby blowing away of the liquid by gushing from the tip of the drain portion 11c is prevented. In the embodiment represented in Figure 5, the pressure relief valve 44a is a three-way solenoid valve, but the pressure relief valve 44a may be a two-way solenoid valve connected on a line branched from the air path as is the relief valve 44b.

The relief valve 44b (see Figure 5) disposed directly downstream the air pump 43 is normally placed in a closed state and carries out pressure relief to the atmosphere when all of the opening and closing valves 45 are closed. The relief valve 44b further relieves the excessively pressurized air by the continuously operated air pump 43 such that the air of a pressure increased during continuous operation of the air pump is prevented from being suddenly supplied into the extraction cartridges 11.

An air-flow path is provided such that the opening and closing valves 45 (represented as a two-way solenoid valve in Figure 5) are individually opened and the pressurized air from the air pump 43 is individually introduced into the extraction cartridges 11 via the corresponding air nozzles 41.

The pressure sensors 46a, which are respectively associated with the air nozzles 41 in one-to-one correspondence, serve to individually detect the internal pressure of the extraction cartridges 11. When the pressure sensor 46a detects that the pressure reaches a predetermined pressure specified as an upper limit of the pressurization (for example, 90 kPa), the detection is transferred to a control unit 8 (see Figure 6). In response, the control unit 8 causes the corresponding opening and closing valve 45 to close in order to stop feeding of the pressurized air, determines whether the pressurization thereto ceases depending on the detection of the reduced pressure associated with the completion of the drainage from the extraction cartridge 11. If determined so, the control unit 8 causes the pressure relief valve 44a to open and controls the system to proceed to the next process.

The pressure sensor 46a serves to detect presence of an extraction cartridge 11 loaded in the cartridge holder 62, presence of a liquid within an extraction cartridge 11, insufficiency of the liquid amount within the extraction cartridge and clogging of the filter, depending on the pressure fluctuations in the extraction cartridge 11. Details will be described later in connection with Figure 9.

The pressure sensors 46b disposed directly downstream the air pump 43 detect the pressure fluctuations, associated with the driving of the air pump 43, in the closed circuit produced when the opening and closing valves 45 and relief valve 44b are closed. The detections provided thereby are used for correcting the air discharge flow rate of the air pump 43 as a part of the driving control of the air pump 43. The details of the correction based on the detection will be described layer.

Figure 6 shows an air supply control system for a single extraction cartridge 11, in which driving control of an air pump 43, actuation of a pressure relief valve 44a and 44b, and individual actuation of an opening and closing valve 45 take place under control of the control unit 8. This control unit 8 controls the pressure to be applied to the extraction cartridge 11, in accordance with a program installed therein, on the basis of a detected pressure signal from the pressure sensors 46a and 46b, an input operation signal from a operation panel 7 disposed at the upper part of the main body 2, and a sample liquid type signal from a data reader 9.

With this configuration, the opening and closing valves 45 are individually opened, thereby introducing pressurized air into the extraction cartridges associated with the valves 45 thus opened. The opening and closing valve 45 in question is closed when the internal pressure detected by the pressure sensor 46a reaches a predetermined pressure specified as an upper limit of the pressurization and then the extraction cartridge 11 is sealed with the inside of the extraction cartridge 11 being pressurized. Subsequently, a pressure is applied to each extraction cartridge 11 to drain the liquid therein through the filter member 11b. Depending on the lowered internal pressure upon completion of the drainage, the pressure sensors 46a respectively check the extraction cartridges 11 associated therewith to detect the cease of the pressurization thereto, i.e., the time of the completion of the drainage thereof. These detections are used for controlling the pressure relief of the individual extraction cartridges by the pressure relief valves 44a.

Further, the control unit 8 serves as means for controlling the flow rate of air to be supplied to the extraction cartridge 11. More specifically, the control unit 8 drives the air pump 43 based on the PWM control or voltage control, and makes corrections such that the flow rate of the air to be supplied to the extraction cartridge 11 falls within a predetermined range based on the pressure measurements by the pressure sensor 46b as means for measuring air flow rate. In this way, the amount and pressure of the pressurized air fed within the extraction cartridge 11 after a predetermined time elapsed from the time when the opening and closing valve 45 is opened.

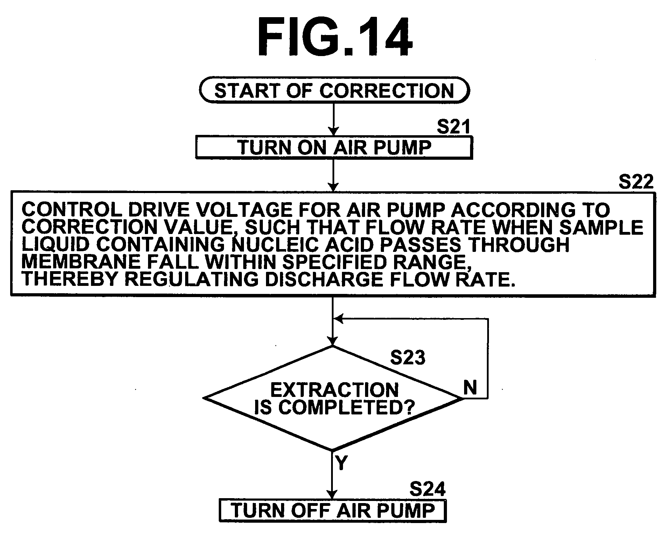

In the pressure measurement performed by the pressure sensor 46b, the air pump 43 is driven at power-on, at the start of each extraction, or at predetermined timings, in order to determine a time period elapsed until a set pressure is reached or to determine a pressure attained after a predetermined time has elapsed, and a correction value (see Figures 12 and 13 described later) is obtained therefrom. Based on the correction value thus obtained, operation of the air pump 43 when operated for extraction is corrected, based on this correction value (see Figure 14 described later). This correction can be performed by manual input or automatic tuning.

Drive control (discharge rate control) for this air pump 43 is performed, for example, based on the PWM control. Specifically, a voltage to be applied to the air pump 43 is varied by appropriately controlling the time ratio (on-off duty cycle control) depending on the number of the extraction cartridges 11 such that the rotational frequency of the air pump 43 is corrected and controlled depending on the correction value increases with increase of the applied pressure, and a rate of pressure rise of the pressurized air to be introduced in the extraction cartridges 11 is made constant, whereby the pressure of the air sealed when the opening and closing valves 45 are closed in response to the detection of the predetermined pressure specified as an upper limit of the pressurization the by the pressure sensor 46a is made constant. The drive control of the air pump 43 may correct the driving voltage according to the correction value.

Further, depending on an input operation signal from the operation panel 7, a sample liquid type signal from the data reader 9 and the like, the control unit 8 changes control parameters including the aforementioned predetermined pressure specified as an upper limit of the pressurization such that operations are performed with the processing procedure and set value most appropriate for the type of the sample liquid S. Specifically, the control unit 8 addresses the change of the type of the sample liquid S to be processed by changing the pressure control method, the amount of the liquid to be processed, and/or the number of washing times, or by additively dispensing a reagent. For example, a protocol, set value and the like which have been programmed beforehand are selected and controlled by, for example, keying in the sample type and the like via a keyboard of the operation panel 7. In the case where different reagents are used for each sample type to be processed, a protocol, a set value and the like which have been programmed beforehand may be selected and controlled, for example, by reading the barcodes or the information on the IC chip attached to the reagents. Similarly, a protocol and/or a set value may be changed by reading a data storage element such as CF card (memory card), on which the information representing the reagent is recorded, by the data reader 9.

In the pressurization, pressurized air may be introduced into a plurality of the extraction cartridges 11 simultaneously or sequentially one-by-one. In order to introduce the pressurized air in the extraction cartridges 11 sequentially one-by-one, one of the opening and closing valve 45, all of which are in their closed positions, is opened to introduce the pressurized air into the extraction cartridge 11 associated with the valve 45 in question. Then, this opening and closing valve 45 is opened according to the detection from the corresponding pressure sensor 46a. Subsequently, the next one of the opening and closing valves 45 is subjected to the procedure of opening the valve 45, introducing the extraction cartridges 11 associated with this valve 45, and closing this valve 45 according to the detection from the corresponding pressure sensor 46a. These procedures are repeated for each of the remaining valves 45. Thus, the pressurized air is introduced into the plurality of extraction cartridges.

Meanwhile, in order to simultaneously introduce the pressurized air into a plurality of extraction cartridges 11, the corresponding number of opening and closing valves 45, all of which are in their closed positions and respectively associated with the plurality of extraction cartridges 11, are opened to introduce the pressurized air into the plurality of extraction cartridges 11. The plurality of the opening and closing valves 45 are individually closed according to the detections from the corresponding pressure sensors 46. In the case where the pressurized air is simultaneously introduced into the plurality of extraction cartridges, the discharge rate of the air pump 43 is controlled, for example, based on the PWM control mentioned above, depending on the number of the opening and closing valves 45 to be opened, such that the rate of pressure rise remains constant.

Figure 15 shows an exemplary control system used for the extraction system according to the fourth embodiment of the invention. the control system comprises, as means for controlling air flow rate, a flow rate control valve 10 (variable orifice) disposed at the suctioning side of the air pump 43. As the structure of the flow rate control valve is similar to that shown in Figure 4 explained earlier, no further description will be given here. The air flow rate control by the flow rate control valve 10 is corrected by the control unit 8 based on the pressure measurement by the pressure sensor 46b in the same way as described above. In this correction, a correction value is calculated based on the pressure detection as shown in Figures 12 and 13 (described later), and the throttle regulation by changing the orifice diameter of the flow rate control valve 10 at the time of extraction based on the correction value is done, thereby correcting and controlling the air flow rate. Note that the aforementioned flow rate control valve 10 may be disposed directly downstream and at the discharging side of the air pump 43

### <Dispensing Mechanism>

The dispensing mechanism 5 includes a washing liquid dispensing nozzle 51w mounted on a nozzle slide 50 which is horizontally movable above the rack 6; a recovery liquid dispensing nozzle 51r, a washing liquid supply pump 52w for feeding a washing liquid W held within a washing liquid bottle 56w to the washing liquid dispensing nozzle 51w, a recovery liquid supply pump 52r for feeding a recovery liquid R held in a recovery liquid bottle 56r to the recovery liquid dispensing nozzle 51r, a waste liquid bottle 57 placed on the holding stage 21, and so on.

The nozzle slide 50 is supported on a guide rail 27 extending horizontally along a vertical wall 26 of the main body 2 and thus laterally movable. The nozzle slide 50 is controlled by a nozzle moving motor (i.e., pulse motor, not shown) such that the nozzle slide 50 is sequentially brought to and stopped at a plurality of positions each correspondingly situated above the respective cartridges 11 and returned as required to a position above the waste liquid bottle 57. The distal end of the washing liquid dispensing nozzle 51w and the distal end of the recovery liquid dispensing nozzle 51r are both bent downward. The washing liquid dispensing nozzle 51w is connected to the washing liquid supply pump 52w via a selector valve 55w, the washing liquid supply pump 52w is connected to the washing liquid bottle 56w via a selector valve 55w, the recovery liquid dispensing nozzle 51r is connected to the recovery liquid supply pump 52r via a selector valve 55r, and the recovery liquid supply pump 52r is connected to recovery liquid bottle 56r via a selector valve 55r. The washing liquid bottle 56w and the recovery liquid bottle 56r are both attached on a side wall of the system main body. The washing liquid supply pump 52w and the recovery liquid supply pump 52r are both composed of a syringe pump. A piston member of washing liquid supply pump 52w is controlled by a pump motor 53w (pulse motor) to dispense a predetermined amount of a washing liquid W according to a position detection from a sensor 54w, while a piston member of the recovery liquid supply pump 52r is controlled by a pump motor 53r (pulse motor) to dispense a predetermined amount of a recovery liquid R according to a position detection from a sensor 54r.

More particularly, in order to dispense the washing liquid W, the selector valve 55w is actuated to select the washing liquid bottle 56w, the pump motor 53w is driven to retract the piston member of the washing liquid supply pump 52w to suction the washing liquid W into the washing liquid supply pump 52w. Subsequently, the selector valve 55w is actuated to select the washing liquid dispensing nozzle 51w, the pump motor 53w is driven to push the piston member of the washing liquid supply pump 52w to cause the washing liquid to be drained through the washing liquid dispensing nozzle 51w into the waste liquid bottle 57 until the air within the passage is discharged, and the washing liquid supply pump 52w is stopped. After that, the washing liquid dispensing nozzle 51w is moved above the extraction cartridge 11, and the washing liquid supply pump 52w is controlled such that a predetermined amount of the washing liquid W is dispensed into the extraction cartridge 11. Meanwhile, in order to dispense the recovery liquid R, the selector valve 55r is actuated to select the recovery liquid bottle 56r, the pump motor 53r is driven to retract the piston member of the recovery liquid supply pump 52r to suction the recovery liquid R into the recovery liquid supply pump 52r. Subsequently, the selector valve 55r is actuated to select the recovery liquid dispensing nozzle 51r, the pump motor 53r is driven to push the piston member of the recovery liquid supply pump 52r to cause the recovery liquid to be drained through the recovery liquid dispensing nozzle 51r into the waste liquid bottle 57 until the air within the passage is discharged, and the recovery liquid supply pump 52r is stopped. After that, the recovery liquid dispensing nozzle 51r is moved above the extraction cartridge 11, and the recovery liquid supply pump 52r is controlled such that a predetermined amount of the recovery liquid R is dispensed into the extraction cartridge 11.

The foregoing control unit 8 (see Figure 6) serves to control not only the pressurization, but also the operations of the foregoing mechanisms 3 to 5. Specifically, the control unit 8 controls a series of processing steps for automatic extraction on the basis of an input operation through the control panel 7 disposed at the upper part of the main body 2 in accordance with a program installed therein.

In the following, the extracting operation of the foregoing extraction system 1 will be described in detail. First, an extraction cartridge 11 is loaded in the rack 6 of the loading mechanism 3, the waste liquid container 12 and the collection container 13 are respectively loaded in the container holder 63, and the rack 6 is placed on the holding stage 21 of the main body. Then, a solubilized sample liquid S is sequentially injected into the extraction cartridges, for example, by a pipette. The sample liquid S may be injected into the extraction cartridges before or after being loaded in the rack 6, prior to the rack being mounted on the system.

Then, when the system is actuated according to an operation of the operation panel 7 by an operator, a lifting and lowering motor 47 of the pressurized air supply mechanism 4 is driven so that the pressing head 40 is moved downward. Accordingly, the tip 49a of the retaining pin 49 is engaged with the pin-receiving hole 62d of cartridge holder 62 and pressing the cartridge holder 62, whereby the cartridge holder 62 is moved downward so as to be positioned in place. At this time, the drain portion 11c located at the bottom of the extraction cartridge 11 is inserted into the waste liquid container 12 to a position spaced a predetermined distance from the leading end of the cartridge as shown in Figure 4, thereby preventing the drained liquid being leaked outside by blowing away or the like. Further, the pressing head 40 is moved downward, whereby the lower end of each air nozzle 41 is brought in sealing abutment against the upper opening of each extraction cartridge 11 via the sealing member 42. Since the retaining pin 49 restricts the position of the cartridge holder 62, each air nozzle 41 can be brought in accurate sealing abutment against the each extraction cartridge 11, whereby a reliable sealing is ensured.

Thereafter, pressurized air is supplied to the extraction cartridges 11. Before the actual supply of the pressurized air takes place, some correction is made such that a predetermined rate of air flow is discharged from the air pump 43. Details of the air flow rate correction will be described in connection with the flow charts shown in FIGS 12 to 14.

The flowchart of Figure 12 shows a routine of calculating a correction value. At power-on or at the start of extraction, the opening and closing valves 45 and relief valve 44b are all closed, thereby producing a closed circuit state. Then, in step S1, the air pump 43 is started. Change of the discharge pressure associated with the driving of the air pump 43 is detected by the pressure sensor 46b, and in step S2, whether the pressure detected by the pressure sensor 46b reaches a predetermined pressure is judged. If the answer in step S2 is NO, the time elapsed to reach the predetermined pressure is measured (step S3).

When the answer in step S12 becomes YES and the predetermined pressure is reached, the time elapsed from the start of the air pump 43 to the time when the predetermined pressure is reached is read, a correction value for controlling the driving of the air pump 43 is calculated depending on the elapsed time in step S4, and the air pump 43 is stopped in step S5. In other words, when the time until the set pressure is reached is long, the discharge flow rate of the air pump 43 is determined to be low, and therefore a correction value is set such that the air discharge rate is increased depending on the degree.

Meanwhile, Figure 13 is a flowchart showing a routine of calculating a correction value, in which the correction value is calculated based on the measurement of the pressure reached after a predetermined time has elapsed. That is, in the same way as described above, in step S11, the air pump 43 is started under the closed circuit state where the opening and closing valves 45 and relief valve 44b are all closed at power-on or at the start of extraction. While determining in step S12 whether the time elapsed from the starting of the air pump 43 reaches a set time, the pressure change of the closed circuit associated with the driving of the air pump 43 is measured by the pressure sensor 46b (S13).

When the answer in step S12 becomes YES and a set time has elapsed, the value of the reached pressure measured is read in step S13, and a correction value for controlling the driving of the air pump 43 is calculated depending on the reached pressure value in step S4, and the air pump 43 is stopped in step S15. In other words, when the pressure reached after a set time has elapsed is low, the discharge flow rate of the air pump 43 is determined to be low, and therefore a correction value is set such that the air discharge rate is increased depending on the degree.

Then, pressurized air is actually supplied to the extraction cartridge 11, and as shown in the flowchart of Figure 14, the air pump 43 is driven in step S21. At this time, in step S22, the drive voltage of the air pump 43 is controlled such that the flow rate at the sample liquid containing a nucleic acid passes through the filter member 11b falls within a specified range, whereby the air discharge rate is controlled.

According to the embodiment with the flow rate control valve 10 shown in Figure 15, the air discharge rate can be controlled by replacing the drive voltage control of the air pump 43 in step S22 described above with the throttle regulation by changing the orifice diameter of the flow rate control valve 10.

When the above-described air pump 43 is driven for extraction from the sample liquid S, a single opening and closing valve 45 is opened, pressurized air from the air pump 43 is supplied to a single extraction cartridge 11 through the air nozzle 41 associated with the valve 45, the pressure within the cartridge 11 is detected by the pressure sensor 46a, and when the pressure thus detected reaches the specified upper limit pressure, the opening and closing valve 45 is closed such that the extraction cartridge 11 is sealed with the inside thereof being pressurized. In the extraction cartridge 11 in which the pressurized air has been introduced, by the action of the pressure, the sample liquid S is passed through the filter member 11b while a nucleic acid is adsorbed to and held on the filter member 11. Other liquid components, which are passed through the filter, are then drained from the lower end of the drain portion of the cartridge into the waste liquid container 12.

Then, the completion of the drainage is detected based on the pressure detected by the pressure sensor 46a. For example, when the acceleration of decrease in pressure reaches or exceeds a predetermined value, the pressure variation associated with the drainage is differentiated twice for observing the change, whereby the pressure fluctuations upon completion of the drainage can be detected accurately regardless of the varying components such as a viscosity of the sample liquid S, a pressurization rate, and the like. Alternatively, the moment that the pressure reduction exceeds a relief pressure (threshold value), i.e. the moment that the pressure difference between the upper limit pressure when the opening and closing valve 45 is closed and the decreased pressure within the cartridge becomes larger than the value thus predetermined is determined as the completion of the drainage. When completion of the drainage from the extraction cartridge 11 is detected based thereon, the pressure relief valve 44a is opened to the atmosphere, and the pressurization to the extraction cartridge 11 terminates. Pressurized air is similarly supplied to each extraction cartridge 11 and the foregoing procedure is repeated for each cartridge until all of the extraction cartridges 11 are pressurized. When completion of the drainage is detected for all of the extraction cartridges by the respective pressure sensors 46a, the pressurization process itself terminates and the pressing head 40 is moved upward.

Figure 9 shows the pressure fluctuations inside an extraction cartridge 11 associated with the introduction of pressurized air into the extraction cartridge 11: curve A represents the characteristics of the internal pressure fluctuation under a normal working condition; and curve B represents the differential waveform representing the change amount thereof. First, the pressure linearly rises from time point "0" (zero) when the opening and closing valve 45 is opened to start introduction of pressurized air. Then, at time point "a" when the pressure reaches a predetermined upper pressure limit (for example, 90 kPa), the opening and closing valve 45 is closed and the internal space of the extraction cartridge is sealed with the inside thereof being pressurized. This pressure acts to pass a liquid within the cartridge through the filter member 11b and decreases with gradual decrease of the liquid inside the cartridge. Subsequently, as the total amount of the liquid is passed through the filter member and thus drainage of the liquid is completed at time point "b", air resistance at the filter member 11b is reduced and the pressure within the cartridge abruptly decreases. The differential waveform curve B exhibits a remarkable pressure fluctuation. Even if the pressure fluctuation does not remarkably appear in the differential waveform curve B, the pressure fluctuation can be made remarkable by differentiating the pressure fluctuation twice. Thus, cease of the pressurization is determined on the basis that the rate of change of pressure reaches or exceeds a predetermined value or that the pressure reduction associated with the drainage from the pressure at point "a" exceeds a predetermined relief pressure (threshold value).

In the case where the sample liquid S is pressurized, the pressure fluctuation differs depending on its viscosity and the like. In the case of a liquid having lower viscosity, the degree of the pressure reduction from point "a" to "b" increases and the time to "b" becomes shorter. When the viscosity of the liquid is relatively high and minor clogging of the filter member 11b occurs, the degree of the pressure reduction from point "a" to "b" decreases and the time to "b" becomes longer.

Further, based on the internal pressure fluctuation characteristics detected by the pressure sensor 46a, detection of a deficient pressurization state, for example, detection of a presence of an extraction cartridge 11 loaded in the cartridge holder 62, detection of the presence of a liquid within an extraction cartridge 11, detection of insufficient sealing, detection of insufficiency of the liquid amount within the extraction cartridge, or detection of clogging of the filter is performed.

First, the deficient pressurization state is detected when a pressure detected by the pressure sensor 46a does not reach a pressure specified for determining the deficient pressurization, for example, as low as 10 kPa. This represents a faulty state where air resistance is low, that is, an extraction cartridge 11 has not been mounted in place, a sample liquid has not been injected, or the sealing between the air nozzle 41 and the extraction cartridge 11 is insufficient.

When the amount of a sample liquid S injected to an extraction cartridge 11 is not the defined amount but a small amount, the initial pressure rises so as to exceed the pressure specified for determining the deficient pressurization mentioned above, but does not rise to the pressure specified as the upper limit for pressurization where the opening and closing valve 45 is closed, and thus the drainage of the liquids is completed before the pressure specified as the upper limit for pressurization is reached, and the pressure abruptly decreases. Such a case is determined as that in which the liquid amount is insufficient.

Further, the case where the detected pressure gradually reduces with the drainage of the liquid, the pressure reduction is small, and after a predetermined time has elapsed, the cease of pressurization upon completion of the drainage cannot be determined and the pressure does not decrease to a value lower than the pressure specified for determining the cease of the pressurization, is determined as that in which clogging of a filter has occurred. Clogging of the filter may be detected by the fact that the state where the decrease of pressure does not reach or exceed a predetermined value is maintained for a time over a predetermined time.

The insufficient pressurization detection when the pressure rise is insufficient during pressurization, the detection of the cease of the pressurization associated with the completion of the drainage, and the detection of the clogging of the filter are similarly carried out during the washing and collection processes described later.

Then, control goes to the washing process, during which raising of the pressing head 40 after the pressurized air is supplied should be carried out with the state shown in Figure 4 being maintained, where the air nozzle 41 has been moved away from the extraction cartridge 11 and brought upward to a position where the movement of the nozzle slide 50 is allowed, and the retaining pin 49 is pressing the cartridge holder 62 such that the lower end of the extraction cartridge 11 is inserted within the waste liquid container 12. Then, the nozzle slide 50 is moved to bring the washing liquid dispensing nozzle 51w to the first extraction cartridge 11, where a predetermined amount of a washing liquid W is dispensed in the first extraction cartridge 11, and then the nozzle slide 50 is brought to the next extraction cartridge 11 for dispensing therein the washing liquid W, and so on. After completion of the dispensing of the washing liquid W to all of the extraction cartridges 11, the pressing head 40 is moved downward, whereby the lower end of each air nozzle 41 is brought in sealing abutment against the upper opening of each extraction cartridge 11 via the sealing member 42. Subsequently, the opening and closing valves are sequentially opened to respectively supply pressurized air into the extraction cartridges 11 in a similar manner as described above. The washing liquid W under pressure passes through the filter member 11b and serves to wash out the impurities other than the nucleic acid adsorbed on the filter member lib. The washing liquid W is drained from the extraction cartridge 11. The pressure relief valve 44a is opened upon completion of the drainage. When the washing liquid W in all of the extraction cartridges 11 has all passed through the filter members 11b and drained, the pressing head 40 is moved upward to its initial position. When the washing process is to be performed more than once, the foregoing procedure is repeated.

The pressurized air used for the washing process and a collection process (described later) may be supplied simultaneously to a plurality of extraction cartridges 11. Specifically, all the extraction cartridges 11 under normal operating conditions, i.e. the extraction cartridges 11 except those having a portion determined as under a deficient pressurized state or a clogged filter portion, are simultaneously opened, pressurized air is introduced therein by the air pump 43 that is variably driven based on the PWM control, and these operating and closing valves 45 are individually closed according to the detections of the upper pressurization limits from the pressure sensor 46a.

Then, control goes to the extraction process. First, an upward movement of the pressing head 50 after the washing process lifts the retaining pin 49, and in turn lifts the cartridge holder 62 of the rack. After the drain portion 11c located at the lower end of the extraction cartridge 11 moved to a position above the waste liquid container 12, the container is replaced with another one by operating the actuating member 31 of the loading mechanism 3 such that the container holder 63 is retracted, and positioning the collection container 13 under the extraction cartridge 11.

Subsequently, the pressing head 40 is moved downward so that the tip of the retaining pin 49 is engaged with the pin-receiving hole 62d of the cartridge holder 62 and presses the cartridge holder 62. Then, the nozzle slide 50 is moved to bring the recovery liquid dispensing nozzle 51r to the first extraction cartridge 11 where a predetermined amount of a recovery liquid R is dispensed in the first extraction cartridge 11, and then the nozzle slide 50 is brought to the next extraction cartridge 11 for dispensing therein the recovery liquid R, and so on. After completion of the dispensing of the recovery liquid R to all of the extraction cartridges 11, the pressing head 40 is moved downward as described above, whereby the lower end of each air nozzle 41 is brought in sealing abutment against the upper opening of each extraction cartridge 11 via the sealing member 42. Subsequently, the opening and closing valves are sequentially opened to respectively supply pressurized air into the extraction cartridges 11. The recovery liquid R under pressure passes through the filter member 11b, serves to desorb the nucleic acid adsorbed on the filter member 11b, and is drained from the extraction cartridge 11. Then, the recovery liquid R is drained together with the nucleic acid into the collection container 13 from the drain port 11c at the bottom end of the cartridge, and the pressure relief valve 44a is opened as described above upon completion of the drainage. After the recovery liquids R within all of the extraction cartridges 11 are all drained into the collection container 13, the pressing head 50 is moved upward, and thus the sequence of operations is terminated.

After completion of such an extracting operation, the rack 6 is dismounted from the holding stage 21, the extraction cartridge 11 and the waste liquid container 12 are respectively removed from the cartridge holder 62 and the container holder 63 and discarded. Meanwhile the recovery container 13 is removed from the container holder 63, capped as required, and subjected to the next nucleic acid analysis or the like.

While in the foregoing description of the embodiment, a plurality of extraction cartridges 11 are mounted on the system, the present invention is not limited thereto. This invention may be applicable to the case where a single extraction cartridge 11 is mounted.

While in the foregoing description of the embodiment, washing by the use of the washing liquid W is performed, a filter member 11b with a certain filtering performance does not necessarily require such a washing process. Although the embodiment was described in relation to a nucleic acid extraction system, the present invention is not limited thereto. This invention is applicable to an extraction system in which various specific materials are brought into contact with a filter member. Further, the specific material need not necessarily be collected by the use of a recovery liquid. The specific material may be analyzed with the specific material being in contact with the filter member or may be analyzed by adding a process liquid to the specific material and examining the resultant color.

## Claims

1. An extraction system for extracting a specific material, comprising:
an extraction cartridge provided with a filter member, wherein a sample liquid containing a specific material is injected into an extraction cartridge and the inside of the extraction cartridge is pressurized to cause the specific material within the sample liquid to be adsorbed to the filter member; and
a pressurized-air supply mechanism for introducing pressurized air into the extraction cartridge,
**characterized in that** the pressurized-air supply mechanism starts to supply the pressurized air to a plurality of extraction cartridge substantially simultaneously, controls the pressure imparted to the filter member within the cartridges at a predetermined pressure, and relieves the pressure after the drainage of the sample liquid within the cartridge is completed.

2. An extraction system as defined in Claim 1, wherein said specified material is a nucleic acid, a recovery liquid is subsequently dispensed into the extraction cartridge, and the nucleic acid is collected together with the recovery liquid.

3. An extraction system as defined in Claim 1 or 2, wherein the pressurized-air supply mechanism includes: an air pump; a feed passage extending from the air pump to a plurality of extraction cartridges; a plurality of opening and closing valves, disposed partway along the feed passage and associated with the plurality of extraction cartridges in one-to-one correspondence, for individually starting or stopping the supply of the pressurized air to the plurality of extraction cartridges; and a plurality of pressure relief valves, disposed partway along the feed passage and associated with the plurality of extraction cartridges in one-to-one correspondence, for individually relieving the pressure within the plurality of extraction cartridges.

4. The extraction system as defined in Claim 2 or 3, wherein the opening and closing valves are independently actuated to individually introduce the pressurized air into the plurality of the extraction cartridges, the plurality of the extraction cartridges are sealed, and upon completion of any one of the drainage of the extraction cartridges, the pressure relief valve associated with the completely drained extraction cartridge is independently opened to relieve the pressurized air remaining in the extraction cartridge.

5. The extraction system as defined in any one of Claims 2 to 4, wherein the pressurized-air supply mechanism further comprises a pressure sensor for individually detecting the internal pressure of the extraction cartridge, and the pressure relief valves are individually opened depending on a detection result provided by the pressure sensor.

6. An extraction system as defined in Claim 1, wherein the pressurized-air supply mechanism including: an air pump; means for controlling air flow rate; and means for measuring air flow rate, wherein the means for controlling air flow rate is controlled depending on a measurement result from the means for measuring air flow rate, such that the flow rate of the air to be supplied to the extraction cartridge falls within a predetermined range.

7. The extraction system as defined in Claim 6, wherein the means for controlling air flow rate is a means for regulating the discharge rate from the air pump by drive-controlling the air pump.

8. The extraction system as defined in Claim 6, wherein the means for controlling air flow rate is a means for restricting the air flow rate at the suction side or discharge side of the air pump by the use of a flow rate control valve.

9. The extraction system as defined in any one of Claims 6 to 8, wherein the means for measuring air flow rate is a means for measuring a pressure in association with driving of the air pump in a closed circuit for air flow.

10. The extraction system as defined in any one of Claims 6 to 9, wherein the air flow rate is corrected by driving the air pump at power-on, at the start of each extraction, or at predetermined timings.
